Europäisches Patentamt

European Patent Office

Office européen des brevets

(19)

(11) EP 0 726 951 B1

(12) EUROPEAN PATENT SPECIFICATION

(45) Date of publication and mention
of the grant of the patent:
**06.10.1999 Bulletin 1999/40**

(21) Application number: **94931655.8**

(22) Date of filing: **04.11.1994**

(51) Int Cl.[6]: **C12N 15/12**, C12N 15/62,
C07K 14/47, C07K 14/435,
G01N 33/68

(86) International application number:
**PCT/GB94/02420**

(87) International publication number:
**WO 95/12672 (11.05.1995 Gazette 1995/20)**

(54) **MANUFACTURE AND USE OF POLYPEPTIDES TAGGED USING BINDING MOLECULES**

HERSTELLUNG UND ANWENDUNG VON POLYPEPTIDEN, DIE DURCH BINDMOLEKUELE MARKIERT SIND

PRODUCTION ET UTILISATION DE POLYPEPTIDES MARQUES AU MOYEN DE MOLECULES DE LIAISON

(84) Designated Contracting States:
**AT BE CH DE DK ES FR GB GR IE IT LI LU MC NL PT SE**

(30) Priority: **04.11.1993 GB 9322772**
**25.03.1994 GB 9405927**

(43) Date of publication of application:
**21.08.1996 Bulletin 1996/34**

(73) Proprietor: **MEDICAL RESEARCH COUNCIL**
**London W1N 4AL (GB)**

(72) Inventors:
• **NERI, Dario**
**ETH Honggerberg CH-8093 Zurich (CH)**
• **WINTER, Gregory, Paul**
**Cambridge CB2 1TQ (GB)**
• **DE LALLA, Claudia, Dibit**
**I-20132 Milano (IT)**

(74) Representative: **Walton, Seán Malcolm et al**
**MEWBURN ELLIS,**
**York House,**
**23 Kingsway**
**London WC2B 6HP (GB)**

(56) References cited:
**WO-A-94/18318**

• **FEBS LETTERS., vol.302, no.3, May 1992, AMSTERDAM NL pages 274 - 278 R E STOFKO-HAHN ET AL. 'A single step purification for recombinant proteins. Characterization of a microtubule-associated protein (MAP-2) fragment which associates with the type II cAMP-dependent protein kinase' cited in the application**
• **JOURNAL OF BIOLOGICAL CHEMISTRY., vol.265, no.35, 15 December 1990, BALTIMORE US pages 21804 - 21810 Z LUO ET AL. 'Identification of MAP-2 and P75-binding domain in the regulatory subunit (RIIbeta) of type II cAMP-dependent protein kinase'**
• **JOURNAL OF BIOLOGICAL CHEMISTRY., vol.264, no.4, 5 February 1989, BALTIMORE US pages 2081 - 2087 T ONO ET AL. 'Molecular cloning sequence and distribution of rat calspermin, a high affinity calmodulin-binding protein'**
• **JOURNAL OF BIOLOGICAL CHEMISTRY., vol.260, no.4, 25 February 1985, BALTIMORE US pages 2527 - 2534 J A COX ET AL. 'The interaction of calmodulin with amphiphilic peptides'**
• **CHEMICAL ABSTRACTS, vol. 116, no. 12, 30 March 1992, Columbus, Ohio, US; abstract no. 123563g, A C RASERA DA SILVA ET AL. 'Hybrid myosin light chains containing a calcium-specific site from troponin C' page 354 ;**

EP 0 726 951 B1

- **JOURNAL OF BIOLOGICAL CHEMISTRY.,
  vol.269, no.17, 29 April 1994, BALTIMORE US
  pages 12594 - 12599 K HAGA ET AL. 'Synergistic
  association of a G-protein-coupled receptor
  kinase by G protein beta-gamma subunits and
  mastoparan or related peptides'**

## Description

The present invention relates to detection, immobilization, targeting and purification of recombinant polypeptides. In particular it relates to the labelling or tagging of proteins with calcium-dependent binding molecules such as calmodulin, for example by creating fusion proteins, enabling use of the resultant molecule in the manners indicated.

A number of different approaches have been taken to general methods of detection, immobilization, targeting and one-step affinity purification for recombinant proteins. The expression in bacteria of antibody fragments derived from monoclonal antibodies (Skerra, A. & Pluckthun,A., 1988 *Science* **240** 1038-1041; Better,M. et al, 1988 *Science* **240** 1041-1043) or from bacteriophage display repertoires (for review, see Winter, G. et al, (1994) *Annual Rev. Immunol.* **12** 433-435) has reinforced the need for a widely applicable detection system. The initial demonstration that chimeric antibodies could be made where antibodies were fused to other proteins which retained their biological function (M.S. Neuberger & T.H. Rabbitts, 1984; UK patent 2,177,096B) opened the way to the construction of such genetic fusions with antibody molecules.

A variety of fusion tags have been used to date for recombinant proteins: (i) a peptide which recognises a specific antibody e.g. the *myc* tag, (Munro, S., and Pelham, H.R.B. (1986) *Cell,* **46**, 291-300; Ward, E.S., Güssow, D., Griffiths, A.D., Jones, P.T. and Winter, G. (1989) *Nature,* **341**, 544-546); the Flag-peptide (Hopp, T.P. et al (1988) *BioTechnology,* **6**, 1204-1210); the KT3 epitope (Martin, G.A. et al (1990) *Cell,* **63**, 843-849; Martin, G.A.,et al (1992) *Science,* **255**, 192-194); an α-tubulin epitope (Skinner, R.H. et al (1991) *J.Biol.Chem.,* **266**, 14163-14166); the T7 gene 10 protein peptide tag (Lutz-Freyermuth, C., Query, C.C. and Keene, J.D. (1990) *Proc. Natl. Acad. Sci. U.S.A.,* **87**, 6393-6397) or an affinity support using poly-histidine tails binding to nickel-chelating agarose (Skerra, A., Pfitzinger, I. and Plückthun, A. (1991) *BioTechnology,* **9**, 273-278; Lilius, G., et al (1991) *Eur. J. Biochem.,* **198**, 499-504); the strep-tag, a peptide tag binding to streptavidin (Schmidt, T.G.M. and Skerra, A. (1993) *Protein Engineering,* **6**, 109-122); (ii) a protein domain which forms a complex with a second (macro)molecule e.g. glutathione-S-transferase (Smith, D. B. and Johnson, K.S. (1988) *Gene* **67**, 31-40); bovine pancreatic trypsin inhibitor, BPTI, (Borijin, M. and Nathans, J. (1993) *Proc. Natl. Acad. Sci. U.S.A.* **90**, 337-341); maltose binding protein, MBP, (Bedouelle, H. & Duplay,P. (1988) *Eur. J. Biochem.* **171** 541-549; Maina, C.V. et al. (1988) *Gene* **74** 365-373), or (iii) a polypeptide sequence that can be biotinylated and thus made to interact with avidin or streptavidin (Schatz, P.J. (1993) *Bio/Technology* **11**, 1138-1143).

Typically, some applications, (such as affinity purification) require a specific but low affinity interaction in order not to impair the function of the recombinant protein with a harsh elution protocol. Other applications (such as targeting and immobilisation) require slow release and high-affinity binding of the tag. The known tag systems described above do not have this flexibility and although they perform well in many applications, each of them has some undesired draw-back.

Those tags recognized by specific monoclonal antibodies require columns which are either expensive or difficult to prepare. In some cases, harsh conditions are necessary to elute the bound protein (e.g. extreme pH values or chaotropic agents), which may impair the activity of the purified protein. Moreover, the affinity of the tag for the monoclonal antibody is usually not high enough for some applications, most notably for a stable immobilization of the tagged protein on a biosensor chip, e.g. for use with the BIAcore (Pharmacia).

The latter point applies also to the Strep-tag and perhaps to the polyhistidine tag, although stable immobilization on metal-chelating support might perhaps be achieved by high-density coating of the matrix. The polyhistidine tag has the draw-back that it does not allow protein detection, useful for example in ELISA or immunoblot.

When the tag is a protein such as the BPTI tag, protein folding requirements of the tag itself, such as disulfide bridge formation, place limits on the possible applications. Furthermore, the high affinity of BPTI for trypsin ($K_d = 6$ x $10^{-14}$ M), makes elution of BPTI-tagged proteins from trypsin difficult.

Stofko-Hahn et al. ((1992) *FEBS Lett.* **302**, 274-278) have made a fusion of a calmodulin-binding peptide tag derived from the C-terminus of rabbit skeletal muscle myosin light-chain kinase and a recombinant protein and used this in purification on an affinity support.

Generally, the present invention provides molecules comprising a binding polypeptide and another polypeptide. There are a number of types of these molecules with various properties making them suitable for various uses, for example in purification of tagged proteins from affinity columns or by ion exchange chromatorgraphy, in band-shift assays and multimerisation. The binding of the binding polypeptide to ligand may be calcium-dependent. By this is meant that the dissociation constant for the ligand is reduced by the presence of calcium ions, that is binding is much stronger in the presence of calcium ions. For the applications described herein, it is preferred that this reduction is at least 10 fold when the binding protein has half of its calcium ion sites occupied. Preferably the dissociation constant is greater than 10nM at a pH of between 6 and 9 at 20°C and 10nM or less in the presence of calcium ions, most preferably 1nM or less. For some calcium-dependent binding proteins, other analagous ions may replace calcium, for example strontium.

In a one aspect the present invention provides a molecule comprising (i) a polypeptide able to bind a ligand with a binding affinity with a dissociation constant ($K_d$) of 10nM or less, measured at a pH of between 6 and 9 at 20°C, and (ii) another polypeptide. Preferably the dissociation constant is 1nM or less. The binding affinity may be calcium-dependent, as discussed. Molecules with these characteristics are especially useful in band shift assays and multimerisation, as discussed further *infra.*

In another aspect the present invention provides a molecule comprising (i) a polypeptide ("calcium-dependent binding polypeptide") able to bind a ligand of calmodulin with a binding affinity which is dependent on the presence of calcium ions, and (ii) another polypeptide. As discussed herein, calmodulin is ideally suited for use in purification of recombinant proteins, binding ligands with high affinity but capable of elution from affinity columns under mild conditions. Other calcium-dependent binding polypeptides able to bind ligands of calmodulin, such as mastoparan and fragments and derivatives thereof, enjoy these same desirable properties and are useful in the same context. An example is troponin C.

For use in band-shift assays (discussed further *infra),* molecules according to the present invention should have not only a low dissociation constant (eg. 10nM or less and preferably 1nM or less, measured at a pH between pH6 and pH9 at 20°C), they preferably have a negative charge at about pH 8 of at least -5, preferably at least -10. The net charge of a protein at about pH 8 is defined by comparing the number of positively charged amino acid residues (lysines and arginines) with the number of negatively charged amino acids (glutamate and aspartate). If there are 10 more negatively charged residues than there are positively charged residues then the net charge is -10.

As discussed, preferred calcium-dependent binding polypeptides are calmodulin and troponin C. Fragments of these, retaining calcium-dependent binding for a ligand, may be used (as demonstrated herein), as may derivatives. The term "derivative" encompasses amino acid sequence variants with, for example, an insertion, deletion or substitution of one or more amino acids compared with wild-type, chimaeric fusion polypeptides and polypeptides modified post-translationally.

Molecules according to the present invention may comprise a binding domain of a member of a specific binding pair (sbp) such as an antibody or other polypeptide comprising an immunoglobulin binding domain. Other sbp members are discussed *infra* and still more will be apparent to those skilled in the art. Properties of binding of the sbp member to complementary sbp member (eg antibody to antigen) may be studied using a band-shift assay (wherein electrophoretic mobility is compared in the presence and absence of complementary sbp member) as provided herein.

For many purposes, molecules according to the present invention may be labelled using, for example, a fluorescer.

For some applications, the calcium-dependent binding polypeptide and ligand need to have a half-life which is long enough to allow employment of a multimer comprising the molecule and ligand. An example of where a reasonably long half-life is required is in a band-shift assay. The components of the multimer must remain bound together in a sufficient amount to allow detection following electrophoresis. Thus, the half-life of the multimer may be a minimum of around 15 minutes, but is preferably at least 60 minutes, at 20°C.

Multimers wherein the ligand is a polypeptide and is fused to another polypeptide may find a number of uses, dependent on the nature of that other polypeptide.

It is preferred that the molecules are produced by expression from nucleic acid comprising gene fusions, such that the components of the molecules are linked by a peptide bond. Nucleic acid encoding such molecules is provided by the present invention: it may comprise a promoter (eg in an expression vector) for expression of the polypeptide fusion within host cells. Host cells containing such nucleic acid may be grown in culture to cause or allow production of the fusion molecules. The "tag" may then be used in the isolation of the resultant recombinant polypeptide, with, eg, elution from an affinity column or ion exchange chromatography. Elution may make use of a calcium-sequestrant/chelator, where the binding polypeptide has calcium-dependent binding affinity for the ligand employed.

Suitable vectors for expression can be chosen or constructed, containing appropriate regulatory sequences, including promoter sequences, terminator fragments, polyadenylation sequences, enhancer sequences, marker genes and other sequences as appropriate. For further details see, for example, *Molecular Cloning: a Laboratory Manual:* 2nd edition, Sambrook et al, 1989, Cold Spring Harbor Laboratory Press. Transformation procedures depend on the host used, but are well known. A variety of different host cells are frequently employed by those skilled in the art, including bacteria, mammalian cells, yeast and baculovirus systems. Mammalian cell lines available in the art for expression of a heterologous polypeptide include Chinese hamster ovary cells, HeLa cells, baby hamster kidney cells, COS cells and many others. A common, preferred bacterial host is *E. coli.*

It should be noted that the term "fusion" is used herein in the context of joining of two polypeptides to form a molecule. The preferred mode of joining is via a peptide bond, so that a "fusion protein" may be produce by expression from encoding nucleic acid therefor. However, other modes of joining are envisaged. The term "fusion" should be understood to cover these unless the context requires otherwise.

Thus, the tag may be appended to another polypeptide by chemical modification or non-covalently. For instance, a cysteine group may be incorporated at the C-terminus of an sbp member such as a single chain Fv antibody fragment and a binding protein such as calmodulin coupled using the heterobifunctional crosslinker SPDP (N-succinimidyl-3-(2-pyridyldithio)propionate). For non-covalent linkage, peptides favouring formation of dimers such as leucine zippers (P. Pack and A. Pluckthun, *Biochemistry,* **31**, 1579-1584, 1992) or fos and jun (J. *Immunol.* **148** 1547-1553, 1992) may be appended to the two polypeptides to be linked. The complex forms on mixing of the proteins. This non-covalent coupling procedure may generate protein-calmodulin complexes which are insufficiently stable for some uses, such as band-shift assays or *in vivo* applications.

In the work leading to aspects of the present invention, we have shown that a number of advantages may arise from linking a $Ca^{2+}$-dependent binding polypeptide, such as calmodulin, to the recombinant protein and using a peptide as a soluble ligand. Thus, a system is provided enabling combination of high affinity binding and mild elution protocols in affinity purification using the binding molecule as a tag, eg for recombinant proteins, in particular recombinant antibodies. Moreover, the high affinity for peptide tags and unusual electrophoretic properties of the calmodulin domain make it suitable for a range of other applications.

Troponin C has suitable properties to be used as an alternative fusion partner instead of calmodulin. It allows mild elution from affinity columns, has a high affinity for peptide tags and has a strong negative charge. Other calcium-dependent binding proteins which are homologous to calmodulin having high affinity for peptide tags are also suitable, eg for mild elution. Suitability may be assessed by determining whether the binding protein binds a ligand of calmodulin, especially a polypeptide ligand of calmodulin such as mastoparan or a derivative of mastoparan.

Such fusions (e.g. with calmodulin) have advantages over other fusion partners in several specific assays. Herein, we disclose the use of recombinant calmodulin fusions in a band shift assay (which may be a gel retardation assay) which allows the study of affinities of recombinant proteins such as antibodies, particularly for protein ligands and the ranking according to affinity of, for example, antibodies expressed from clones from an antibody library. The use of polypeptide fusions in confocal microscopy and fluorescent activated cell sorting is also illustrated. Moreover, the interaction between calmodulin (and other calcium-dependent binding polypeptides, as discussed) and peptide ligands provides a means of heterodimerisation of proteins, as illustrated by the assembly of an antibody-calmodulin fusion with a protein (maltose binding protein) tagged with a peptide ligand of calmodulin. Production of fusions of calmodulin and recombinant proteins by expression from encoding nucleic acid is also disclosed.

Dimerisation, whether formation of homo- or heterodimers, or even heteromulimerisation may be used for instance in tumour cell killing or immunoscintigraphy applications.

Calmodulin is a small protein (148 aminoacids), extremely well conserved between species and poorly immunogenic (VanEldik, L.J. & Lukas, T.J. (1987) *Methods Enzym.* **139**, 393-405). It is very resistant to denaturation, even though it does not contain any disulfide bridge. Human calmodulin has a net 24 negative charges in the absence of calcium or 16 negative charges after calmodulin binding to four calcium ions. Several proteins, peptides and organic compounds bind to calmodulin with nanomolar or subnanomolar affinity (Vorherr, T., et al. (1993)*Biochemistry* **32**, 6081-6088;Ikura, M., et al (1992)*Science,* **256**, 632-638; Fisher, T.H. et al. (1987) *Biochemistry* **26**, 8024; Benitez-King, G., et al (1993) *Life Sciences* **53**, 201-207) in a process that can be inhibited by calcium chelators such as EGTA.

The small molecules which bind to calmodulin include calmidazolium and melatonin. Melatonin has a dissociation constant of 188pM for calmodulin, but despite this high affinity is unsuitable for applications which require its complex with calmodulin to remain intact, such as band-shift assays and multimerisation. The low half-life of dissociation of melatonin from calmodulin means that no complex is detected on band shift assays (Example 11). Thus, a long half life of dissociation, in excess of the time required to perform the experimental procedure required, such as band shift assays, is an essential property of the detection complex in certain contexts.

Peptide tags such as those derived from mastoparan bind to calmodulin with suitable long half lives. The TAG peptides of Table 2 Have off-rates of less than $10^{-4}$ $s^{-1}$.

Herein we also disclose a number of peptides which have not been used previously as tags for binding to recombinant calmodulin. Although these peptides, for example mastoparan have been shown to bind to calmodulin as free peptides, it was not obvious that they would bind in fusions since in the free form they were C-terminally amidated and had a free N-terminus. Mastoparan is the preferred peptide from which to derive designs for tag peptides. However, there are other peptides which bind with slightly lower, but nevertheless high affinity to calmodulin on which designs can be based, for example melittin, SK-MLCK, NO-30 and AC-28 (Table 1).

There are advantages arising from the unusual charge properties of calmodulin outlined above. The highly negatively charged calmodulin moiety causes the chimaeric protein to migrate towards the anode. The calmodulin tag allows the recombinant fusion proteins to be purified on anion exchange resins, and also direct the fusions to the anode in native polyacrylamide gels over a wide range of pH values facilitating gel retardation assays (D.W. Carr & J.D. Scott *Trends Biochem. Sci.* **17** 246-250, 1992; P. Ramanujam et al *BioTechniques* 8 556-563, 1990). This has particular advantages when screening for example antibodies from phage libraries where the consistency of migration of the calmodulin fusions means that band shift assays can be set up directly to monitor supernatants from bacterial cultures as in example 7, without necessitating purification. We also demonstrate that despite calmodulin being an intracellular protein, it can be expressed in a fusion with antibody fragments by secretion from bacteria.

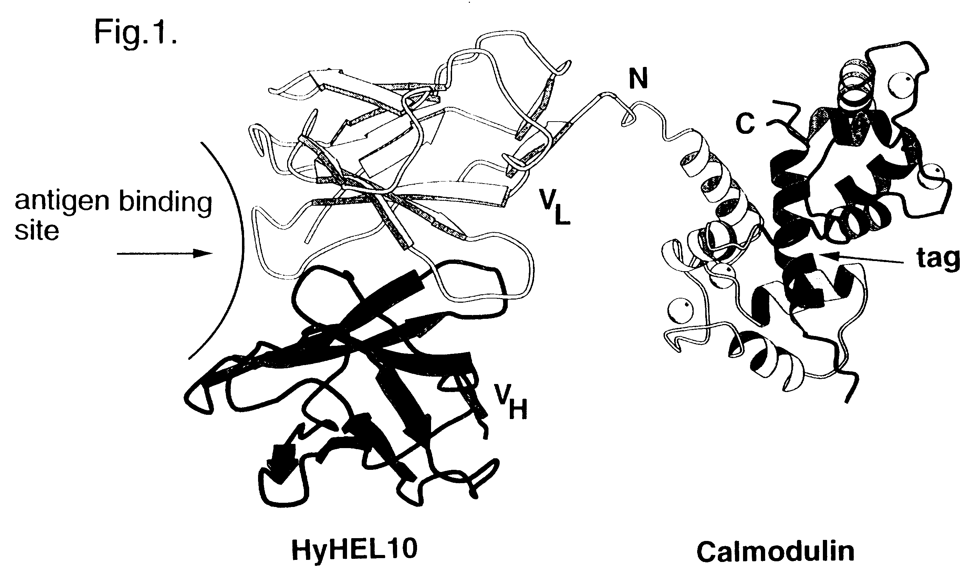

In designing an antibody-antigen fusion, a computer model of an antibody-Fv fragment fused to calmodulin was built from the known three dimensional structures of the antibody HyHEL10 (directed against hen egg lysozyme) and calmodulin. The scFv fragment of HyHEL10 and calmodulin were linked by a two residue peptide (Ala)$_2$ between the C-terminus of the VL domain and the N-terminus of calmodulin (Figure1) to place the binding sites for antigen and calmodulin ligands far enough apart to avoid interference between the sites. The similar three-dimensional structure of homologous proteins such as troponin C means they can be fused in a similar manner.

Calcium-dependent binding proteins suitable for use in various aspects of the present invention include calmodulin, troponin C, parvalbumin and oncomodulin.

[It should be noted that, unless the context requires otherwise, the term "antibody" is used herein to refer to whole antibody, any functional antibody fragment, or any polypeptide comprising an immunoglobulin binding domain, whether natural or synthetic. It has been shown that the function of binding antigens can be performed by fragments of a whole antibody. Example binding fragments are (i) the Fab fragment consisting of VL, VH, CL and CHI domains; (ii) the Fd fragment consisting of the VH and CH1 domains; (iii) the Fv fragment consisting of the VL and VH domains of a single antibody; (iv) the dAb fragment (Ward, E.S. et al., Nature 341, 544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site; (viii) bispecific single chain Fv dimers (PCT/US92/09965) and (ix) diabodies, multivalent or multispecific fragments constructed by gene fusion - these are multimers of polypeptides, each polypeptide comprising a first domain comprising a binding region of an immunoglobulin light chain and a second domain comprising a binding region of an immunoglobulin heavy chain, the two domains being linked (eg by a peptide linker) but unable to associate with each other to form an antigen binding site: antigen binding sites are formed by the association of the first domain of one polypeptide within the multimer with the second domain of another polypeptide within the multimer (WO 94/13804).]

Fusion with a $Ca^{2+}$-dependent binding molecule (e.g. calmodulin) can provide a handle for purification of polypeptides such as antibodies or fragments thereof by anion exchange chromatography or by affinity chromatography on (e.g. calmodulin) antagonist columns. The fusions can be eluted under mild conditions with high salt from ion-exchange, or mildly with EGTA from calmodulin antagonist affinity columns. Other calcium chelators/sequestrants may be used for elution, for example EDTA (ethylenediaminetetraacetic acid) which is less selective than EGTA (ethylene glycol bis (β-aminoethylether)-N,N,N',N'-tetraacetic acid).

A site for cleavage by a specific protease may be incorporated between the recombinant protein and the calmodulin or other binding molecule. For instance, a site for cleavage by Factor Xa (EP 161937) or enterokinase (EP 035384) may be incorporated. This provides means for cleaving the recombinant protein from (e.g.) calmodulin if required for final application of the recombinant protein. Further it would also be possible to release recombinant protein from a calmodulin-ligand affinity column by treatment with Factor Xa (likewise other binding molecule fusion partners).

Furthermore the binding moiety can be readily detected by pre-incubation with fluorescent peptide ligands, as illustrated by the imaging of fluorescent bands on gel electrophoresis, and cell surface antigens by confocal fluorescence microscopy and fluorescent activated cell sorting (FACS). In addition to fluorescein exemplified herein, other fluorescent reporter groups may be used, for example 7-nitrobenz-2-oxa-1,3 diazole (NBD), Texas red, coumarin or rhodamine.

Other highly sensitive methods for detection of calmodulin and other tags may be developed, for example by $Tb^{3+}$ luminescence after replacing $Ca^{2+}$ ions with $Tb^{3+}$ ions as recently described for oncomodulin (Clark, I.D., MacManus, J.P., Banville, D. & Szabo, A.G. (1993) *Anal. Biochem.* **210**, 1-6). The preparation of a fusion protein between a single chain Fv fragment with oncomodulin has been reported with the purpose of enabling the labelling of the complex with $Tb^{3+}$ ions (R. Mackenzie et al, 4th IBC Conference on Antibody Engineering, San Diego, December 1993, Abstract 45).

Calcium-dependent binding proteins other than calmodulin may be used in systems disclosed herein, as long as they are able to bind labelled tags (such as the labelled TAG peptides of Table 2). For instance, troponin C, C-terminal fragments of calmodulin or mutants (amino acid sequence variants) of calmodulin may be used. Indeed, it has been shown that the TAG peptide binds well to troponin C (Example 10). Troponin C has a net negative charge of -27 at neutral pH and thus will tend to make fusions migrate towards the anode. Other calcium binding proteins, which may be homologous to calmodulin, such as calcineurin, oncomodulin and parvalbumin, may be screened for ability to bind suitable molecules such as mastoparan derived tag peptides, and their electrophoretic behaviour examined to determine their suitability for use in band shift assays. As discussed, for use in such assays high affinity binding and an off-rate such that there is not significant dissociation during electrophoresis are required.

C-terminal fragments of calmodulin (eg amino acids 81-148) bind mastoparan-derived peptide tags and may be used in, for example, band-shift assays, as shown in Example 10. Mutants, derivatives and variants of calmodulin and other binding molecules may be used as long as they retain binding affinity for the tag (eg mastoparan). The C-terminal domain of troponin C (amino acids 94-162) may be usable. In contrast, the N-terminal domain of calmodulin does not bind the TAG peptides (Example 10).

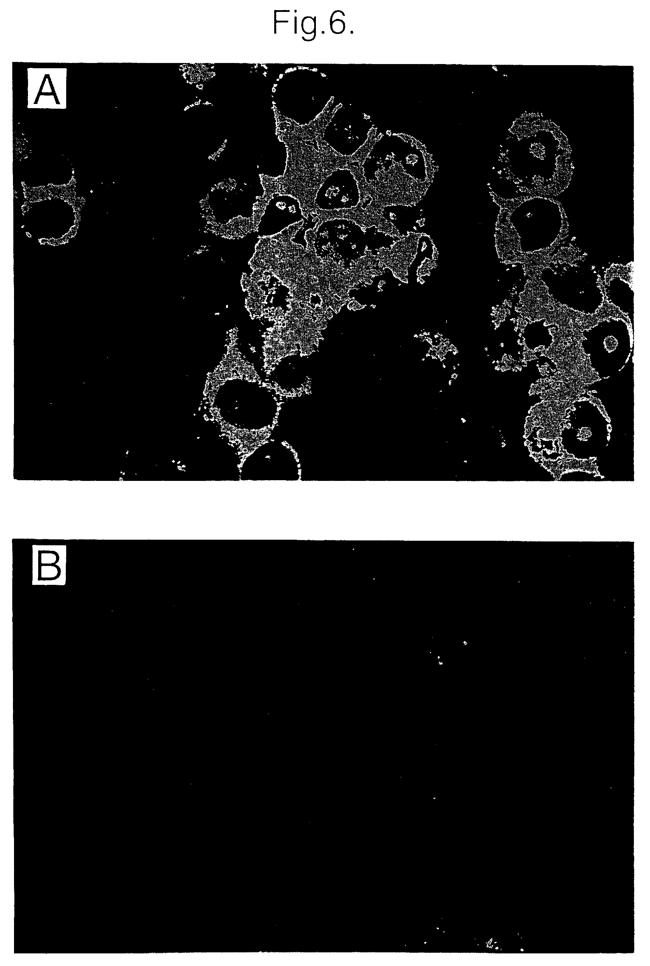

Calmodulin fusions allow convenient detection of complexes, eg antibody-antigen, by band shift on gel electrophoresis (Figure 5), a technique originally employed for studying protein-DNA interactions (Müller, M., et al. (1988) *Embo J.* **7**, 4299-4304; Carey, J. (1988) *Proc. Natl. Acad. Sci. U.S.A.* **85**, 975-979). The highly acidic nature of calmodulin (net charge -25 at pH 7.0) helps ensure that (for example) both the antibody fusion and the complex with antigen move toward the anode, even with highly basic antigens such as HEL as shown in example 7 (Figure 5). A highly sensitive fluorescent gel imager allows the detection of subnanomolar concentrations of the fusion protein, and its complex with antigen (Figure 5). Complex formation is even detectable using calmodulin fusions in bacterial extracts (Figure 5). However, the antigen-antibody complexes presumably need to be stable (as with HyHEL-10 and MFE-23), with half-lives longer than the time taken for gel electrophoresis (15 min). The half-life of the complexes between calmodulin and the peptide tags described in this invention are well in excess of that needed for the native gel electrophoresis.

The band shift assay system, where the use of calmodulin fusions and peptide tags allows study of the relative affinity of members of specific binding pairs, such as antibodies with antigens, may be used with other fusion partners and labelled detection polypeptides. For example, the calmodulin/mastoparan pair may be replaced with thrombin/hirudin (T.J. Rydel et al, *Science* 1990, **249**, 277-280; E. Skrzypczak-Junkun et al, *J. Mol. Biol.* 1991, **221** 1379-1393); bovine pancreatic trypsin inhibitor/trypsin (Borijin and Nathans, 1993, *supra)* or barnase/barstar (Buckle et al, *Biochemistry* 1994, **33** 8878-8889). It is preferable that the fusion partner for the antibody defines the electrophoretic characteristics of the antigen in a similar way to calmodulin, due to having a negative charge of at least -5 at about pH 8 and preferably greater than -10 at about pH 8. The fusion partner should have a high affinity for its complementary tag and there is a slow-off rate for the binding interaction so that the enough complex remains intact throughout electrophoresis.

Thus, troponin C is also a suitable fusion partner to use with mastoparan-derived tags. Hirudin, like calmodulin, provides a negatively charged partner which favours migration towards the anode. Hirudin, however, has the disadvantage compared with calmodulin that the hirudin contains disulphide bonds which are likely to reduce the efficiency of expression in bacteria. In contrast, it is shown herein that calmodulin fusions can be expressed efficiently in bacteria. Further, the hirudin/thrombin has the disadvantage that the thrombin molecule is much larger (288 amino acids) than calmodulin, making it more difficult to distinguish complexed from uncomplexed sbp members. Possible tags for this system include hirudin (65 amino acids) hirulog 1 (20 amino acids) and hirulog-1 (12 amino acids).

Thus, a scheme for band shift assay may be written as follows:

$$A\text{-}C + B^* \rightarrow A\text{-}C(B^*)$$

Complex migrates at position x on electrophoresis

$$A\text{-}C + B^* + D \rightarrow A\text{-}C(D)(B^*)$$

Complex migrates at position y on electrophoresis

Affinity of C for D is related to $\frac{[A\text{-}C(D)\,(B^*)]}{[A\text{-}C(B^*)]}$ where C and D represent specific binding pair (sbp) members under study (eg C is an antibody fragment and D is an antigen), A is the fusion partner for the sbp member under study (eg calmodulin), A-C is the fusion polypeptide, B is the molecule which binds the fusion partner, eg a mastoparan-derived peptide tag. $B^*$ is B labelled, eg, with fluorescein.

Alternatively, A could be labelled ($A^*$) and B unlabelled.

Hirudin/thrombin are alternatives for A and B and peptide hormones/receptors are alternative sbp members C and D which could be studied.

The principles of the use of binding molecule fusion in the study of antibody-antigen binding pairs is applicable also to other specific binding pairs, for instance peptide hormones/receptors, transcriptional activators/DNA binding domains and cell surface signalling molecules such as T-cell receptors/MHC complex.

Thus, a further aspect of the invention provides a band-shift assay method for determining binding affinity of the binding domain of a member of a specific binding pair for a complementary sbp member of interest, the method comprising binding a binding polypeptide of a molecule according to the invention to a labelled ligand and comparing electrophoretic mobility of the resultant multimer in the presence and absence of the complementary sbp member. The binding polypeptide should be able to bind ligand with a binding affinity with a dissociation constant ($K_d$) of 10nM or less, measured at a pH of between 6 and 9 at 20°C. The binding affinity may be dependent on the presence of calcium ions. As discussed, the half-life of the multimer is preferably at least about 15 minutes, most preferably at least about 60 minutes.

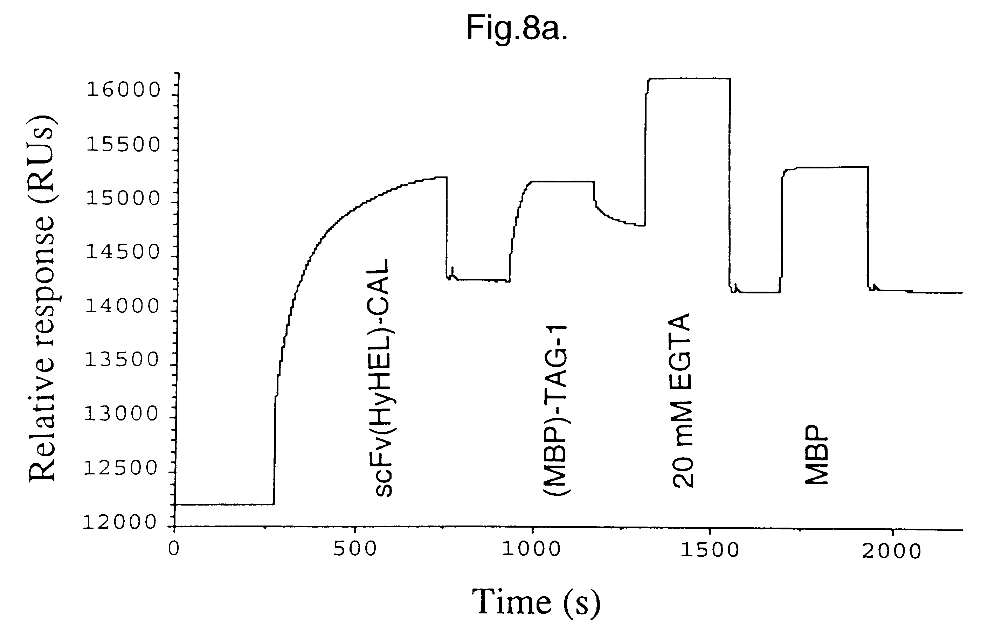

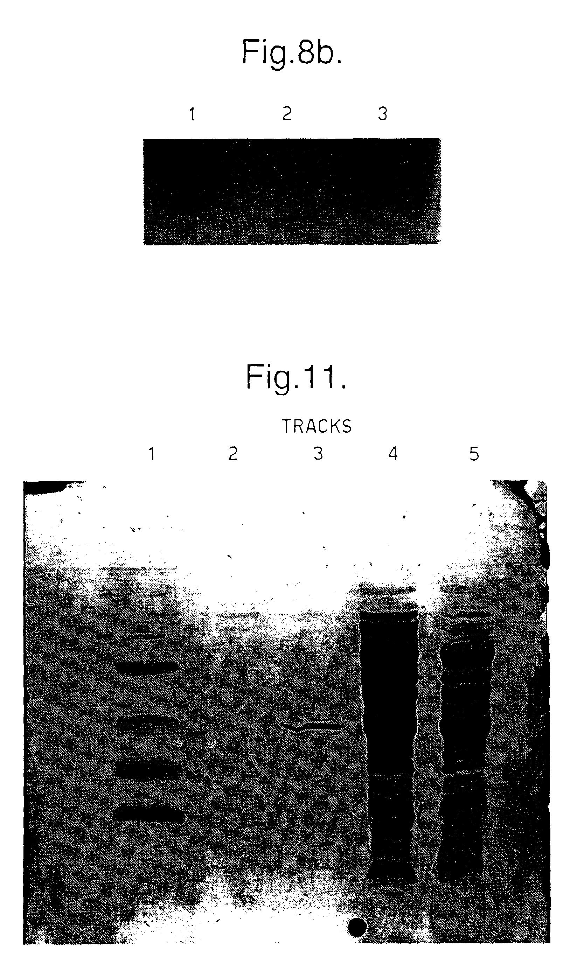

The strong binding interactions between calmodulin and peptide ligands also provide a means of creating dimers, or higher multimers. Dimeric peptide ligands were found to dimerise the antibody-calmodulin fusions (not shown); presumably multimeric peptide ligands would be capable of producing antibody multimers. Peptide tags were also used to create heterodimers; thus antibody fused to calmodulin was able to associate with MBP tagged with the calmodulin-binding peptide (Example 9; Figure 8). Although the tag was stable when attached to MBP (expressed inside the bacteria), it was rapidly proteolysed when appended to secreted antibody fragments. For some applications it may therefore be desirable to design protease resistant tags.

This might be done by fusing derivatives of peptides where random amino acids have been incorporated at potential cleavage sites, for instance the two amino acids KK. Thes variable peptides could be fused to a single chain Fv and displayed on phage (WO92/01047) and phage could then be selected by binding to calmodulin. Peptide tags which are more resistant to protease activity and retain affinity for calmodulin may be selected. The phage display process should enrich for these.

Thus, a still further aspect of the invention provides multimers as disclosed herein, with a binding polypeptide fused to another polypeptide and the binding polypeptide non-covalently bound to ligand. The ligand may be a polypeptide fused to another polypeptide.

Antibody-calmodulin fusions have potential for *in vivo* use. Calmodulin is poorly immunogenic (VanEldik & Lukas, 1987 supra.), and it has been shown that calmodulin is not toxic, does not accumulate selectively in any organ and is rapidly excreted in the urine. In principle, for immunoscintigraphy antibody calmodulin fusions could be targeted to tumour cells, and detected by radioactively labelled calmodulin ligands. Indeed small specific ligands appear to detect tumours well (Kalofonos, H.P., (1990) *J. Nucl. Med.* **31**, 1791-1796; Paganelli, G., et al. (1991) *Cancer Res.* **51**, 5960-5966). A tag system as disclosed herein may be used instead of the established avidin-biotin system. Alternatively the antibody calmodulin fusion could be used to recruit effector functions to the tumour, for example another antibody fragment binding to cytotoxic T lymphocytes.

For *in vivo* applications, for example, multimeric complexes may be formed. For instance, to recruit cytotoxic T lymphocytes to tumour sites and antibody-calmodulin fusion directed against a tumour may be complexed with an antibody-mastoparan peptide fusion, and used for *in vivo* tumour killing. Alternatively, a two-stage process may be used wher the anti-tumour fusion is injected and binds to the tumour and subsequently the anti-T cell fusion is added. If the second fusion is instead labelled with an isotope a similar system may be used for immunoscintigraphy.

In addition to use of calmodulin or other ligand-binding polypeptide as a "tag" in the numerous ways disclosed herein, the format may be reversed so that the "tag" is a calmodulin-binding polypeptide (which may be referred to as a peptide). The polypeptide may be mastoparan or a polypeptide derived from mastoparan. Preferred such polypeptides are shown in Table 2. Derivatives which retain calmodulin-binding ability may be used. Thus, mastoparan or a mastoparan derived polypeptide may be joined, eg by a peptide bond, to another polypeptide, which may comprise a binding domain of a member of a specific binding pair (eg an immunoglobulin binding domain) and used in purification, isolation, labelling, assaying and other ways disclosed herein and discussed at length *supra* eg for "tagging" with calmodulin.

Various aspects and embodiments of the present invention are illustrated in the following examples with reference to the figures. Further aspects and embodiments of the present invention will be apparent to those skilled in the art.

All documents mentioned in the text are incorporated by reference.

**Brief description of the figures**

**Figure 1: HEL in complex with HyHELIO Fv fragment fused to calmodulin**. Modelled by MOLSCRIPT (Kraulis, P. (1991) *J. Appl. Cryst.* **24**, 946-950).

**Figure 2: Cloning vector pDN152 for the expression of the scFv-CAL fusions**. rbs, ribosome binding site; Pel B, leader sequence.

**Figure 3: Purification of scFv-CAL fusions**. Lanes: (M) markers (Sigma); (1) supernatant HyHEL-10; (2) periplasmic MFE-23; (3) HyHEL-10 purified on HEL-Sepharose (4) MFE-23 purified on N-(6-aminohexyl)-5-chloro-1-naphtalene-sulfonamide-agarose; (5) MFE-23 purified on anion exchange Mono-Q.

**Figure 4: Dissociation of peptide ligands from calmodulin**. 600 nM calmodulin was pre-mixed in TBSC and 10% sucrose with 50 nM fluorescein-labeled TAG3 peptide. Aliquots were incubated on ice with a large excess (7 μM) unlabeled peptide for 0, 0.5, 1, 2, and 4 hrs, and analysed on a native 15% polyacrylamide gel (as in Laemmli, 1970 supra except with $CaCl_2$ to 1 mM) in a Hoefer Mighty Small apparatus, and imaged with a cooled CCD-camera based fluorescent gel imaging system developed in collaboration with Confocal Technologies (Liverpool, UK) ard Digital Pixel (Brighton, UK).

**Figure 5: Binding of scFv-CAL fusions to antigen by fluorescent band shift assay.** 1 μl of periplasmic extracts or purified antibody were incubated with 1 μl of 10 μM fluorescinated TAG-3 peptide, 8 μl TBSC or with 8 μl 100 μM antigen solution in TBSC. The samples were run on a Homogeneous 20 Phast Gel (Pharmacia) using native buffer strips, and the gel imaged. Lanes: (1) periplasm extract MFE-23; (2) purified MFE-23; (3) purified MFE-23 and CEA; (4) purified MFE-23 and HEL; (5) periplasm extract HyHEL-10; (6) purified HyHEL-10; (7) purified HyHEL-10 and HEL; (8) purified HyHEL-10 and CEA (see below for details).

**Figure 6: Binding of scFv-CAL fusions to cell surface antigen by confocal fluorescence laser microscopy**. LOVO cells (surface CEA) incubated with TAG3 peptide in complex with (a) scFv(MFE-23)-CAL or (b) scFv (HyHEL-10)-CAL.

**Figure 7: Binding of scFv-CAL to MKN45 cells (surface CEA) detected by FACS using fluorescein-labeled TAG3 peptide**. (a) scFv(MFE-23)-CAL; (b) scFv(HyHEL-10)-CAL.

**Figure 8a: Binding of scFv-CAL and MBP-TAG1 detected by surface plasmon resonance (Pharmacia Biacore)**. 2500 resonance units (RUs) of turkey egg lysozyme (TEL) (Sigma) were immobilised on a microsensor chip of a BIAcore machine (Pharmacia). A solution of scFv(HyHEL-10)-TAG1 (5 μg/ml) in TBSC was injected onto the chip (flow rate 5 μl/min), followed by injections of MBP-TAG1 (5 μg/ml), then 20 mM EGTA (to dissociate the complex) and then MBP (New England Biolabs 5 μg/ml).

**Figure 8b: Homogeneous 20 Native Phast Gel (Pharmacia) for the analysis of scFv(HyHEL-10)-CAL and MBP-TAG1 interaction**. Conditions as in Figure 5, except the gel was stained with Coomassie. Lanes: 1, MBP-TAG1 (0.3 mg/ml); 2, scFv (HyHEL10)-CAL (0.08 mg/ml); 3, scFv (HyHEL-10) (0.08 mg/ml) + MBP-TAG1 (0.3mg/ml). The scFv (HyHEL10) band shows retarded mobility upon addition of an excess of MBP-TAG1.

**Figure 9: Fluorescent and Coomassie blue detection of fluorescent peptide/ calmodulin complexes**.

**Figure 10: Map of vector pDN123**.

**Figure 11: SDS-polyacrylamide gel on samples from the purification of TAG-1 tagged scFvD1.3 from pDN124**.

**Figure 12: amino acid sequence of calmodulin**.

## Example 1: Evaluation of calmodulin-binding peptides as tags.

**Table 1 shows the alignment of homologous peptide sequences which bind to calmodulin with high affinity ($K_a > 5 \times 10^7$ M$^{-1}$; Ikura et al., 1992, et. supra; Malencik, D.A. and Anderson, S.R. (1983) *Biochem. Biophys. Res. Comm.*, **114**, 50-56). Amongst them, mastoparan has the shortest sequence (14 amino acid residues) and the highest affinity ($K_a = 3.3 \times 10^9$ M$^{-1}$), and was the best candidate for the development of the calmodulin binding peptide tag.

**The structure of the calmodulin/SMLK-peptide (Ikura et al., 1992, et. supra) was used to model mastoparan binding to calmodulin. The homology between mastoparan and SMLK-peptide sequences (Table 1) and preliminary NMR data, which revealed that mastoparan makes contacts with both N-terminal and C-terminal domain of calmodulin. The structure is reminiscent of a stick (mastoparan) in a dough-nut (calmodulin).

**Some potential problems became immediately apparent from the mastoparan sequence (Table 1), if it is to be used as a tag. Firstly, the mastoparan C-terminal carboxyl group is amidated. It was necessary to ascertain whether mastoparan in the deamidated form (i.e. with a free carboxylate group at the C-terminus) still binds to calmodulin with high affinity, and whether binding is still preserved when the C-terminus is extended with other peptide sequences. If this were the case, then mastoparan could be used as N-terminal tag, fused to the protein of interest. To use the tag at the C-terminal extremity of the protein it is necessary to ascertain whether the positively charged -NH$_3^+$ moiety could be replaced by a foreign peptide sequence.

## Example 2: Evaluation of the requirement for free N- and C-termini in the mastoparan-derived tag

**Four peptide analogues of the mastoparan sequence were synthesized to investigate the requirement for free N- and C-termini in binding to calmodulin. These are shown in Table 2. All contain a cysteine as functionalization site and the C-terminal sequence of antibody V$_L$ domain (EIKR) followed by the AAA tripeptide encoded by the *Not*I restriction site sequence, followed by mastoparan. The four peptides have different C-termini: -COOH, -CONH$_2$ and two peptide sequences derived from the calmodulin-binding peptide melittin.

**The four peptides TAG-1 to TAG-4 were fluoresceinated, then complexed with a slight excess of calmodulin and run into a native gel. The peptide-calmodulin complex formation was observed both by fluorescent imaging of the gel (detecting peptide) and by coomassie-blue staining (detecting calmodulin). Comigration of fluorescent peak and coomassie-blue stained band indicates the formation of a stable peptide-calmodulin complex.

Peptide synthesis and modification.

**Solid phase peptide synthesis was carried out on a model 350 multiple peptide synthesizer (Zinsser Analytic, Frankfurt, Germany), using Fmoc/t-butyl protecting group chemistry. Removal of the Fmoc groups was by treatment with 20% (v/v) piperidine in dimethylformamide and successive amino acid residues were added as N-hydroxybenzotriazole esters. Side-chain deprotection and cleavage from the resin was by 93% trifluoroacteic acid/3% 1,2-ethanediol/2% thioanisole/2% water. Peptides were tested for homogeneity by resolution on a Vydac C18 column (10mM, 100 x 250mm) and checked by amino acid analysis (PICO TAG, Waters, Milford, MA, USA).

Peptide fluoresceination

For peptide fluoresceination, lmg of each peptide was dissolved in 1ml of 100mM NaHCO3, pH 8.9. One volume of peptide was mixed with one volume of water and 2 volumes of 2mM iodoacetamidofluorescein (Molecular Probes Inc.) in dimethylformamide. The reaction was allowed to proceed for 15 minutes then quenched by adding dithiothreitol to 10mM final concentration. The fluoresceinated peptides were separated from unreacted fluorescein using a Millipore Mem-Sep cartridge column (TBS = loading and wash buffer; TBS + 0.5 M NaCl = elution buffer).

Native gel electrophoresis.

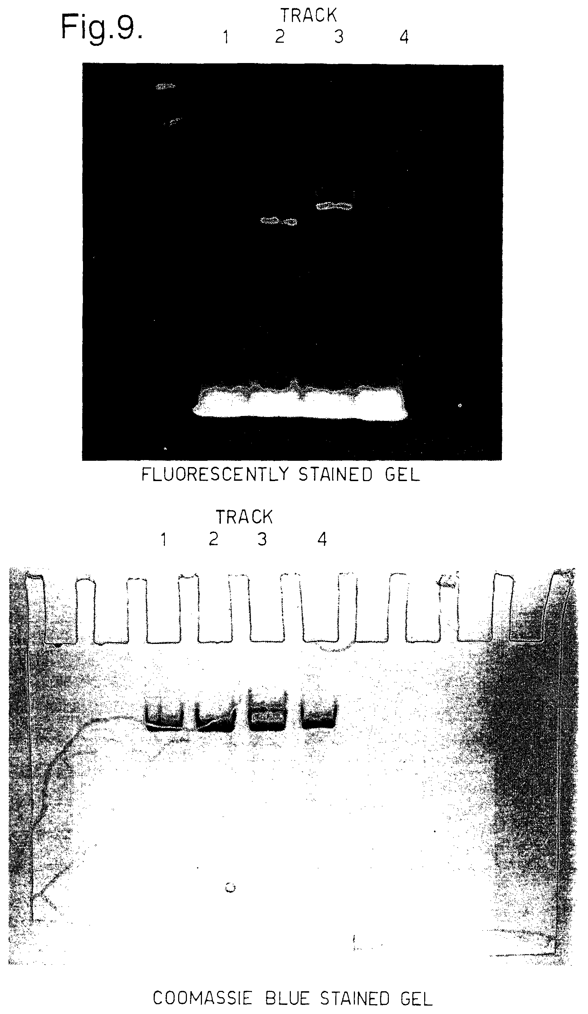

A 20μl reaction was set up containing ca. 0.4μg of fluoresceinated peptide and 18μg of calmodulin (Sigma) in TBS containing 1mM $CaCl_2$ (TBS is 50mM Tris-HCl, pH 7.4, 100mM NaCl in water). 7μl of native gel mix (50% glycerol + 0.05% bromophenol blue) were added, and 8μl of each reaction resolved on a 15% native polyacrylamide gel at 150V, 15°C. The gel was first imaged on a U.V transilluminator and photographed, then stained with coomassie blue and photographed again.
The result is shown in figure 9. Tracks 1, 2 & 4 show calmodulin complexed with TAG-1, 2 & 4 respectively. The fluorescent and coomassie blue tracks coincide, indicating the presence of peptide in stable complex with calmodulin. In track 3 (TAG-3 + calmodulin) two bands are evident on coomassie staining and it is the upper of these that has bound peptide wheras the lower is free calmodulin. The mobility of the TAG-3 + calmodulin complex differs from the previous two samples because of the positively-charged C-terminal extension; the free calmodulin band is visible because calmodulin is provided in excess in this experiment.
From this experiment, it was concluded that both N- and C-termini of the peptide sequence can be extended without preventing binding to calmodulin. This indicates that the mastoparan derived tags are an appropriate ligand for detection, purification and immobilisation of macromolecules, whether placed at either end or within the molecule of interest.

**Example 3: Purification of antibody single-chain Fv fragment fused with TAG peptide using calmodulin-agarose.**

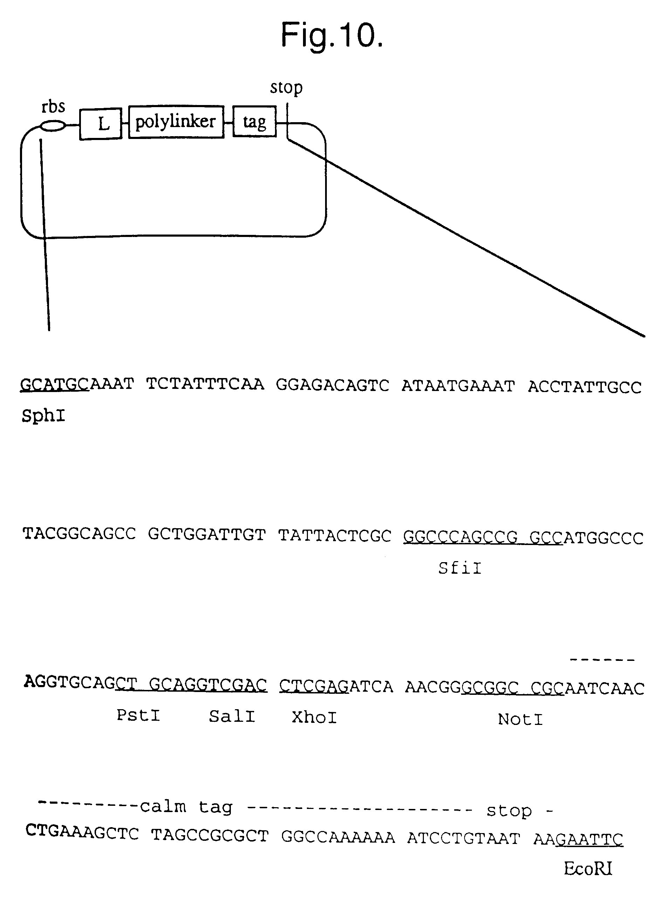

This example describes the construction of genetic fusions between mastoparan-derived TAG peptide tags and recombinant proteins and their purification on calmodulin agarose.
The expression vector pDN123 depicted in Figure 10 was constructed. This vector was designed for expression of soluble proteins in E.coli, appending mastoparan-derived TAG peptides at the C-terminal extremity of the protein of interest. The peptides incorporated are AAA-INLKALAALAKKIL when cloning at the NotI site and LEIKR-AAA- AAA-INLKALAALAKKIL when cloning at the Xhol site. Single-chain Fv antibody fragment (scFv) encoding the anti-Hen egg-white lysozyme antibody D1.3 was cloned into this vector as an Sfi I-Not I fragment to produce plasmid pDN124 and transformed into *E.coli* TG1.

Growth and expression of soluble antibody.

TG1 cells containing plasmid pDN124 were grown overnight at 30°C in 50 mls of 2x YT medium (2x YT is per litre; 16g Bacto-tryptone, 10g bacto-yeast extract, 5g NaCl) containing 1% glucose and 100μg/ml ampicillin. This culture was added to 500ml of fresh medium of the same composition in a 2.5 litre glass conical flask and grown with rapid shaking for 1hour at 30°C. The cells were then pelleted by centrifugation at 5,000x g, 10°C for 10 minutes, the supernatant decanted and the cell pellet resuspended in 500ml of 2x YT containing 1mM IPTG and 100μg/ml ampicillin. The cells were returned to the 2.5 litre conical flask and grown with rapid shaking for 3hours at 30°C.

Extraction of periplasmic proteins.

Cells were pelleted as above, and resuspended in 10 ml of PBS (PBS is per litre of water: 8g NaCl, 0.2g KCl, 1.44g $Na_2HPO_4$, 0.24g $KH_2PO_4$, adjusted to pH 7.4 with HCl) containing 1mM EDTA. After 15 minutes incubation on ice the extracts were centrifuged at 10,000x g, 4°C for 15 minutes, and the supernatant recovered. $CaCl_2$ was added to 10mM final and the extract centrifuged at 20,000x g, 4°C for 15 minutes, and the supernatant applied at a rate of 1ml/min to a 10ml bed volume calmodulin-agarose column (Sigma) pre-equilibrated in TBS + 1mM $CaCl_2$. The column was washed firstly with 20mls of TBS + 1mM $CaCl_2$ then with the same buffer made to 0.4M with NaCl until the optical density of the flowthrough at 280nM was less than 0.01. The antibody was eluted TBS + 1mM EGTA. Yield of scFv was ca. 5mg from 500mls.

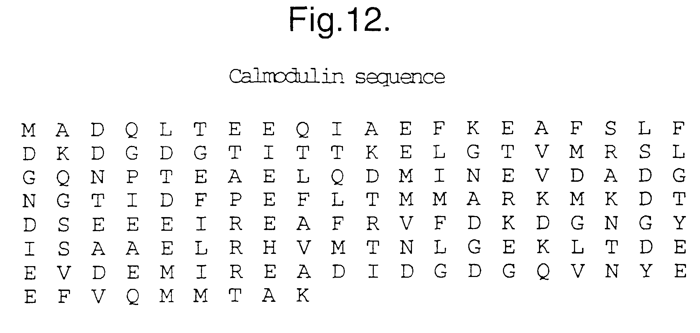

Figure 11 shows a SDS-polyacrylamide gel of the results of the purification of scFv(D1.3) from pDN124. Lane 1 shows the molecular weight calibration marker bands (size expressed in kiloDaltons). In lane 4 and 5, periplasmic preparations of scFv(D1.3) without and with the C-terminal TAG peptide tag were run. The corresponding protein(s) retained on calmodulir agarose column and subsequently eluted, were run in lane 2 and 3. It is evident that the system allows the one-step purification of proteins fused with the TAG peptides (lane 3) from complex protein mixtures (lane 5).

**Example 4 Binding of calmodulin to the scFv-TAG peptide fusions as detected using BIACore**

A BIACore microsensor chip (Pharmacia) was coated with hen egg lysozyme to high surface density. scFv (D1.3) anti-lysozyme antibody fragments fused to the TAG peptide were purified on calmodulin columns or from periplasmic extracts were then examined for calmodulin binding. Both calmodulin-purified antibody and antibody from periplasmic extracts showed typical D1.3 lysozyme binding behaviour. In each case, calmodulin (in Tris buffered saline containing 0.1mM $CaCl_2$) was passed over the chip. Calmodulin binding to the TAG peptide fusion was observed and the apparent off-rate from the lysozyme/TAG peptide fused scFv (D1.3) surface was measured.

**Example 5 Cloning expression and purification of antibody-calmodulin fusions**

In the work described in this example, calmodulin is fused to a recombinant protein and this is a target for a peptide ligand. Calmodulin fusions are purified by affinity and ion exchange chromatography.

**Cloning.**

We constructed an expression vector that allows cloning of the desired gene (in our case recombinant antibody fragments) into restriction sites in the vector polylinker, appends at the C-terminal extremity a gene coding for residues 2-149 of Xenopus laevis calmodulin (which encodes the same protein sequence as human calmodulin) and directs the expression of the resulting chimaeric protein into the periplasmic space of *E.coli* cells.

The gene for the <u>Xenopus laevis</u> calmodulin (Chien, Y. & Dawid, I. (1984) *Mol. Cell. Biol.* **4**, 507-513; provided by C. Klee) was amplified by PCR (1 min at 94°C, 1 min. at 60°C and 2 min at 72°C for 25 cycles) using the primers 5' AGT TCC GCC ATA GCG GCC GCT GAC CAA CTG ACA GAA GAG CAG 3' and 5' ATC CAT CGA GAA TTC TTA TCA CTT TGA TGT CAT CAT TTG 3' to append a *Not*I site, two stop codons and an EcoRI site. The product was digested with NotI and EcoRI, and cloned into pHEN1 (Hoogenboom, H.R., et al. (1991) *Nucl. Acids Res.,* **19**, 4133-4137) to give the pDN152. The genes encoding the scFv antibody fragments HyHEL-10 (anti-lysozyme; Lavoie T.B.,et al. (1992) *J. Immunol.* **148**, 503-513) and MFE-23 (anti-carcinoembryonic antigen, anti-CEA; Chester, K.A.,et al. (1994) Lancet **343**, 455-456) were subcloned into the Sfi1/Not1 sites of pDN152, to give pDN162 and pDN154, respectively, which were transformed into E. coli TG1 (Gibson T J (1984) University of Cambridge) for expression of soluble antibody and purification (see below). The anti-lysozyme antibody fragment scFv D1.3 was also subcloned into pDN152.

Further single chain Fv fragments have been cloned into pDN152, expressed and purified. These are a scFv fragment derived from a hybridoma expressing B1, a murine monoclonal antibody that is an anti-idiotype to the surface Ig of the lymphoma BCL-1 (J. Brissinck et al *J. Immunol.* <u>147</u> 4019-4026, 1991) and 225-28S a scFv fragment directed against melanoma cells.

**Expression, detection and purification of calmodulin fusion proteins.**

Cultures of TG1 harbouring pDN154 or pDN162 were grown at 37°C overnight (in 2xTY, 100 μg/ml ampicillin, 1% glucose), diluted 1: 100 in fresh medium (2xTY, 100 μg/ml ampicillin, 0.1% glucose) to $A^{600} = 0.8$, induced at 22°C with 1mM IPTG and grown overnight. Culture supernatants and periplasms were prepared as described by Schmidt and Skerra (1993; supra) and assayed by ELISA. 96 wells plates (Falcon) were first coated with purified CEA (10 μg/ml in PBS; gift of J. Embleton) or hen egg lysozyme (3 mg/ml in PBS;Sigma) overnight and then blocked with 2% milk powder in PBS (200 μl/well 2 hs). 100 μl of antibody calmodulin fusion was added to each well, followed by 15 μl anti-calmodulin Fab fragment DN169/F3 (Griffiths, A.D., et al. (1994). Isolation of high affinity human antibodies directly from large synthetic repertoires. *EMBO J.,* in the press; 50 μg/ml) and 15 μl anti-human $C_k$ horseradish peroxidase conjugate (The Binding Site; 1:40 dilution) in 2% milk powder in PBS. After incubation at room temp for 15 min, the plate was washed 4 times with PBS and developed with BM-Blue (Boehringer Mannheim) chromogenic substrate.

**Purification of MFE23-calmodulin fusion**

The 50 ml periplasmic extract from a 1 litre culture of pDN154 was made up to 20 mM $CaCl_2$ and affinity purified. The sample was loaded on a 3 ml column of N-(6-aminohexyl)-5-chloro-1-naphtalenesulfonamide-agarose (Sigma) in TBSC (TBS + 1 mM CaCl$_2$), and washed with TBSC + 0.5 M NaCl. The sample was eluted with 20 mM EGTA, and made up to 50 mM CaCl$_2$. Alternatively, the periplasmic extract was filtered and purified by ion exchange (in the absence of calcium) by FPLC using a Mono-Q 5/5 anion exchange chromatography column, washing with TBS and eluting with TBS and a NaCl gradient (0 to 1M). The fusion protein eluted at 0.5 M NaCl. The expression level and the purification yields were checked by 20% SDS polyacrylamide PHAST gel (Pharmacia).

**Expression and purification of scFvHyHEL10-calmodulin fusion protein.**

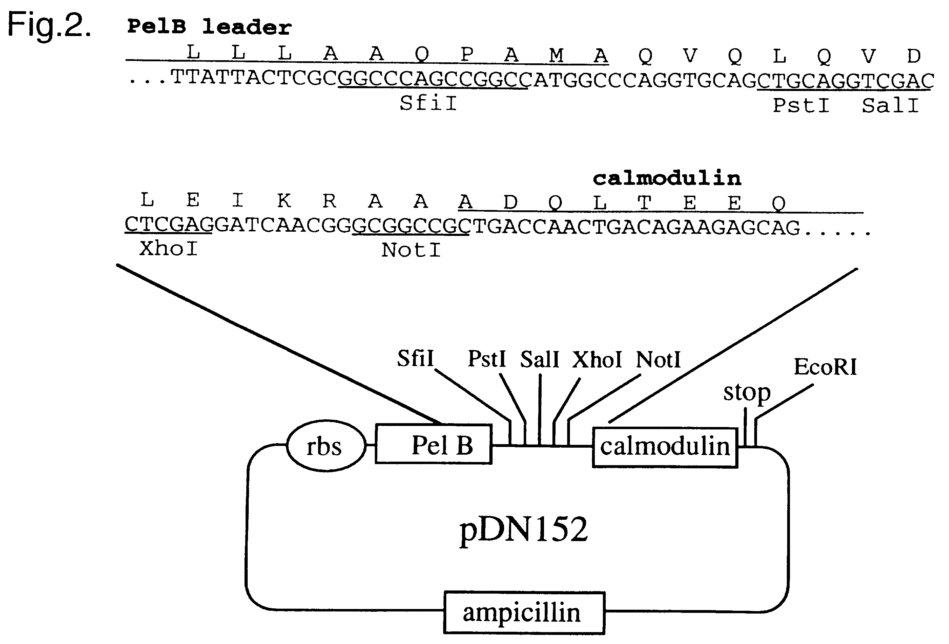

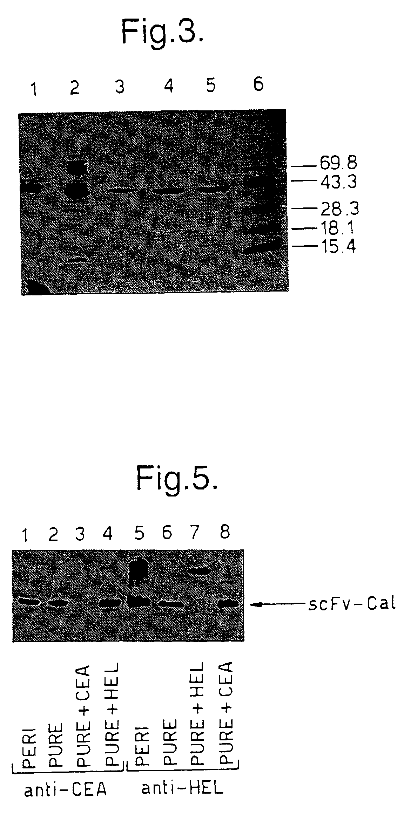

The gene encoding the scFv fragment of the anti-lysozyme antibody HyHEL-10 (Lavoie et al., 1992; supra) was cloned into a vector (pDN152) to append the calmodulin gene as described above and give the clone pDN162 (Figure 2), and the fusion protein expressed by secretion from bacteria. The formation of active antibody could be assayed by the presence of a positive ELISA signal. The fusion protein could be purified from the culture broth or from periplasmic lysates by affinity chromatography on HEL-Sepharose (Ward et al, supra). The yield of the fusion protein from shaker flasks was about 15 mg/l culture (Figure 3), similar to the levels of expression of the scFv fragment (not shown).

The fusion protein could also be purified to homogeneity with similar yields in a single step using the calmodulin ligand N-(6-aminohexyl)-5-chloro-1-naphtalenesulfonamide-agarose (binding with calcium, eluting with EGTA), or by anion-exchange chromatography (using FPLC on a column of Mono-Q resin (Pharmacia).

These experiments indicate the utility of calmodulin tags for affinity purification, and suggest that both the antibody and calmodulin moiety were folded and functional.

**Expression and purification of scFvD1.3-calmodulin fusion protein.**

The single chain FvD1.3- calmodulin fusion was shown to bind lysozyme by the ELISA procedure above. Surface plasmon resonance analysis of scFv(D1.3)-calmodulin fusion protein binding to lysozyme was performed on a BIAcore machine (Pharmacia), immobilizing 2000 RUs lysozyme on a microsensor chip, subsequently injecting scFv (D1.3)-calmodulin supernatant and observing specific binding to the chip and an off rate of 0.003 s$^{-1}$ typical of the D1.3 antibody.

The chimaeric antilysozyme antibody scFv (D1.3) fused to calmodulin was purified to homogeneity using either a lysozyme-Sepharose affinity column or a N-(6-aminohexyl)-5-chloro-1-napthalenesulfonamide-agarose resin. In the latter case purification was easy and mild. Periplasmic extracts containing scFv(D1.3)-calmodulin, washing with TBS with 1mM CaCl$_2$ (TBSC), then with TBSC plus 400mM NaCl and finally eluting with TBS with 20mM EGTA. The product was pure as judged by SDS-PAGE and gave rise to a band in native gels which was shifted by the addition of an excess of lysozyme to the sample. Typically, several milligrams of fusion protein were obtained from 1 litre of culture.

Thus, fusions of calmodulin with single chain Fv fragments can be readily expressed and purified under mild conditions.

**Example 6 Determination of dissociation properties of calmodulin with fluorescent peptides and detection of calmodulin with fluorescent peptides.**

We created fluorescent peptide ligands of calmodulin by modifying mastoporan, a peptide that binds calmodulin with high affinity (K$_d$ = 3 x 10$^{-10}$ M; Malencik and Anderson, 1983; supra; the sequence of mastoporan was extended at both the N- and C-terminus (Table 2), and an N-terminal cysteine residue introduced for labelling with iodoacetamidofluorescein. The synthesis of the fluorescent peptides was as described in example 2.

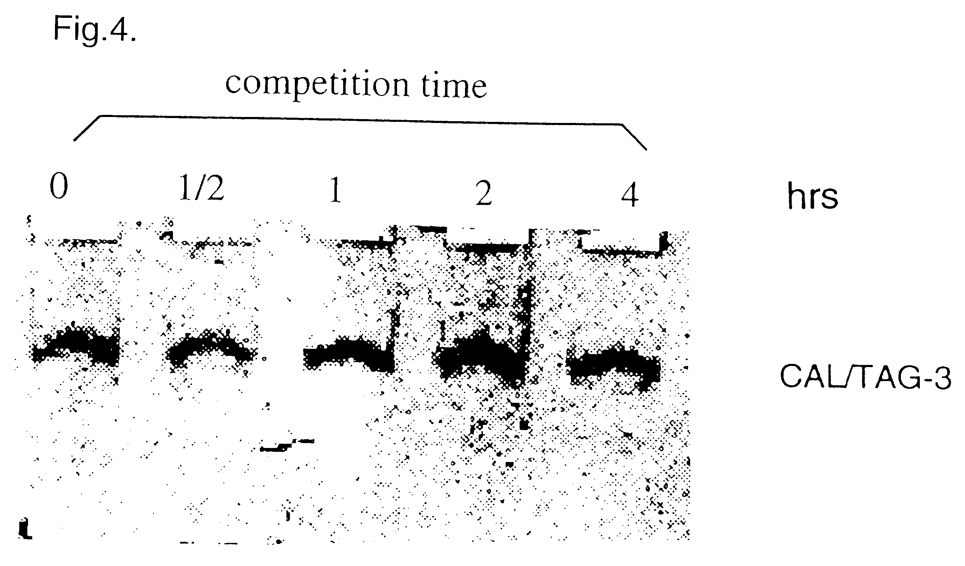

The fluorescent peptides were shown to form stable complexes with calmodulin (Figure 4), with slow off-rates. 600 nM calmodulin was pre-mixed in TBSC and 10% sucrose with 50 nM fluorescein-labeled TAG3 peptide. Aliquots were incubated on ice with a large excess (7 μM) unlabelled peptide for 0, 0.5, 1, 2, and 4 hrs, and analysed on a native 15% polyacrylamide gel (as in Carr & Scott, 1992 supra) except with CaCl$_2$ to 1 mM) in a Hoefer Mighty Small apparatus, and imaged with a cooled CCD-camera based fluorescent gel imaging system developed in collaboration with Confocal Technologies (Liverpool, UK) and Digital Pixel (Brighton, UK).

Thus, when the complex of calmodulin with fluorescent TAG3 peptide was incubated with a large excess of unlabelled peptide, and the mixtures analysed on a native polyacrylamide gel using a fluorescent gel imager, the fluorescent peptide was not significantly displaced from the calmodulin even after four hours competition.

**Example 7 Use of antibody-calmodulin fusions in band shift assays**

The scFv-calmodulin fusions HyHEL10 and MFE23 generated above with specificity for lysozyme and CEA respectively were shown to bind to their antigens by ELISA and were then shown to be effective in band shift assays.

The fluorescent peptides generated in example 2 were then used to detect the formation of antigen-antibody complexes. Thus hen egg lysozyme (HEL) was added to the fluorescently labelled anti-lysozyme scFv (HyHEL-10)-calmodulin fusion, and the mixtures were analysed by gel electrophoresis. The peptides could be used to fluorescently label single chain Fv fragment-calmodulin (scFv-CAL) fusions from both purified preparations or periplasmic extracts (Figure 5) to give a bandshift assay.

1 μl of periplasmic extracts or purified antibody were incubated with 1 μl of 10 μM fluorescinated TAG-3 peptide, 8 μl TBSC or with 8 μl 100 μM antigen solution in TBSC. The samples were run on a Homogeneous 20 Phast Gel (Pharmacia) using native buffer strips, and the gel imaged. Lanes: (1) periplasm extract MFE-23-CAL; (2) purified MFE-23-CAL; (3) purified MFE-23-CAL and CEA; (4) purified MFE-23-CAL and HEL; (5) periplasm extract HyHEL-10-CAL; (6) purified HyHEL-10-CAL; (7) purified HyHEL-10-CAL and HEL; (8) purified HyHEL-10-CAL and CEA.

A "band shift" was seen, indicating formation of a complex; the completeness of the shift at concentrations of HEL > Kd for scFv (HyHEL-10) indicates that all of the fusion (as detected with the fluorescent peptide) binds to HEL. As expected, a band shift was not detected on adding HEL to a fluorescent calmodulin fusion (anti-CEA scFv (MFE-23)) with different antigen specificity (Figure 5). However the band did shift on adding the cognate CEA; indeed the band disappeared entirely, presumably due to the high molecular weight (and aggregation) of CEA.

It is of interest that both scFv-calmodulin fusions and the complex with lysozyme move towards the anode, even with highly basic antigens such as HEL. Complex formation could even be detected using calmodulin fusions in bacterial extracts (Figure 5). However the antigen-antibody complexes presumably need to be stable (as with HyHEL-10 and MFE-23), with half-lives longer than the time taken for gel electrophoresis (15 min). The half-life of the complexes between calmodulin and peptide tags are well in excess of that needed for the native gel electrophoresis.

## Example 8 Use of antibody-calmodulin fusions in confocal microscopy and FACS studies

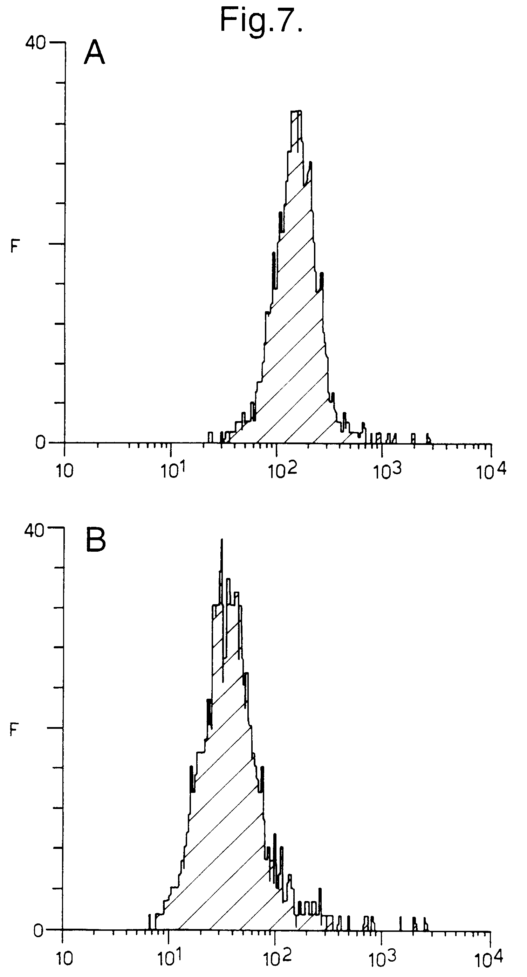

The fluorescent antibody-calmodulin fusions could also be used as immunological reagents; the scFv(MFE-23)-calmodulin fusion was also used to detect CEA on the surface of a cell line LOVO by confocal laser microscopy (Figure 6), and by FACS analysis with the cell line MKN45 (Figure 7).

## Cell targeting

Confocal Laser Microscopy was used to study the use of calmodulin fusions in cell targeting. LOVO cells (ATCC designation CCL 229), expressing the carcinoembryonic antigen (CEA) on their membrane, were grown on microscope glass cover slips using RPMI and 10% fetal calf serum as culture medium. When the cells had grown almost to confluence the cover slips were immersed in acetone, then washed with TBSC and incubated with scFv (MFE-23)-calmodulin periplasmic extracts or with TBSC (negative control) for 30 minutes. The cover slips were then washed for 1 minute with TBSC, then incubated with fluorescinated peptide TAG-3 (1 μM; see Table 2) for 2 minutes, finally washed for 2 minutes with TBSC and analysed with an MRC-600 confocal laser microscope. Figure 7 shows binding of scFv-CAL fusions to cell surface antigen by confocal fluorescence laser microscopy. LOVO cells (expressing surface CEA) were incubated with TAG3 peptide in complex with (a) scFv(MFE-23)-CAL or (b) scFv(HyHEL-10)-CAL.

## FACS.

MKN45 cells (gift from J. Embleton), expressing CEA on their membrane, were grown using RPMI, 10% fetal calf serum as culture medium. Cells were detached form the flask after 20 min. incubation in PBS, 2 mM EDTA with a cell scraper, centrifuged (10 min, 800 rpm) and resuspended in TBSC at a final concentration of $10^6$ cells/ml. 5 μl fluoresceinated TAG3 peptide ($10^{-5}$ M) were added to 50μl antibody-calmodulin fusion ($10^{-5}$ M scFv(HyHEL-10)-CAL; or 2 x $10^{-6}$ M scFv(MFE-23)-CAL), and after five mins the cells (500 μl) were added and incubated on ice for 20 min. The cells were then centrifuged (5 min, 1200 rpm), resuspended in 1 ml TBSC and analysed on a Becton & Dickinson FACScan. Figure 7 shows binding of scFv-CAL to MKN45 cells (surface CEA) detected by FACS using fluorescein-labeled TAG3 peptide: (a) scFv(MFE-23)-CAL; (b) scFv(HyHEL-10)-CAL.

## Example 9 Assembly of bifunctional antibody-calmodulin fusions

The interaction of calmodulin and peptide suggests a means of assembling bifunctional macromolecules, as illustrated here by appending the peptide ligand as a tag to the C-terminus of the maltose binding protein (MBP). The maltose binding protein (MBP) gene was cloned into a pUC119-derived cytoplasmic expression vector (pDN124), which appends the TAG1 peptide (and the protein purified on amylose resin).

After expression in bacteria, and purification on an amylose affinity column, the tagged MBP was shown to interect with antibody calmodulin fusion using surface plasmon resonance (Jönsson. U., et al. (1991)*BioTechniques* **11**, 620-627). Thus HEL was immobilised on the microsensor chip, and the chip saturated with with scFv(HyHEL-10)-CAL. The tagged MBP (but not MBP alone) was shown to bind to the chip, and could be dissociated by addition of EGTA (Figure 8). The scFv calmodulin fusion and tagged MBP were also able to form a stable complex as shown by gel electrophoresis (not shown).

Figure 8a. shows binding of scFv-CAL and MBP-TAG1 detected by surface plasmon resonance (Pharmacia Biacore). 2500 resonance units (RUs) of turkey egg lysozyme (TEL) (Sigma) were immobilised on a microsensor chip of a BIAcore machine (Pharmacia). A solution of scFv(HyHEL-10)-TAG1 (5 µg/ml) in TBSC was injected onto the chip (flow rate 5 µl/min), followed by injections of MBP-TAG1 (5 µg/ml), then 20 mM EGTA (to dissociate the complex) and then MBP (New England Biolabs 5 µg/ml). The scFv calmodulin fusion (scFv-CAL) and the tagged MBP (MBP-TAG1) were also able to form a stable complex as shown by gel electrophoresis (Figure 8b). Figure 8b shows a Homogeneous 20 Native Phast Gel (Pharmacia) for the analysis of scFv(HyHEL-10)-CAL and MBP-TAG1 interaction. Conditions were as in Example 7 and Figure 5, except the gel was stained with Coomassie. Lanes: 1, MBP-TAG1 (0.3 mg/ml); 2, scFv (HyHEL10)-CAL (0.08 mg/ml); 3, scFv (HyHEL-10) (0.08 mg/ml) + MBP-TAG1 (0.3mg/ml). The scFv (HyHEL10) band shows retarded mobility upon addition of an excess of MBP-TAG1.

**Example 10 Band shift assays using alternative fusion partners to the complete calmodulin molecule**

In order to be a suitable fusion partner for use in a bandshift assay, a molecule or a fragment of a molecule must have a high affinity for the ligand (eg tag peptide) and an off-rate low enough that the complex remains intact throughout the electrophoresis. In order to define further the types of calcium-binding proteins which may be used in these off-rate studies, in this example the properties of fragments of calmodulin and of the homologous calcium binding protein troponin C were examined.

C-terminal fragments of calmodulin (amino-acids 81 to 148) and N-terminal fragments (amino acids 1 to 80) were fused to single chain Fv D1.3 (anti-lysozyme) and expressed essentially as in Example 5. 1µl of purified scFv (D1.3)-CAL (Example 5), scFv(D1.3)-CAL(N-domain), scFv(D1.3)-CAL(C-domain), calmodulin or troponin C were incubated with 10µM fluoresceinated TAG-3 peptide and 8µl TBSC. The samples were run on a High-Density Phast Gel (Pharmacia) using native buffer strips, and gel imaged. Complex formation with TAG-3, shown by detection of a fluorescent band, was observed for all the proteins except for scFv(D1.3)-CAL(N-domain).

Thus the C-terminal domain of calmodulin and troponin C are both potential fusion partners for the analysis of the affinity behaviour of a member of a specific binding pair.

**Example 11 Assessment of melatonin as a potential detection molecule for calmodulin-fusion molecule**

The high affinity of melatonin for calmodulin ($K_d$ = 188pM) suggested it might be a useful detection molecule for fusions of calmodulin with recombinant proteins.

In order to assess this, 1µl calmodulin (lmg/ml) was incubated with 1µCi 2-[$^{125}$I]-melatonin in 9µl TBSC and run on a 15% polyacrylamide gel. The gel was as the running gel of Laemmli *(Nature* 1970 **220** 680-685), but with 0.5mM $CaCl_2$ and no SDS in both gel and buffer. The gel was then autoradiographed, but no radioactive band was detected, indicating that the complex dissociates too rapidly, in line with the published half-life of the calmodulin-melatonin complex (120s; Benitez-King et al., 1993).

Thus the half-life of the detection complex is a crucial parameter in determining the suitability of a fusion partner and a labelling tag, especially for use in a band-shift assay.

TABLE 1:

Sequences and affinities of calmodulin-binding peptides

| NAME | | Kd |
|---|---|---|
| Ca++ pump | QILWFRGLNRIQTQIRVVNAFRSS-NH$_2$ | 10 nM |
| SK-MLCK | KRRWKKNFIAVSAANRFKKISSSGAL | 1 nM |
| Melittin | GIGAVLKVLTTGLPALISWIKRKRQQ | 3 nM |
| Mastoparan X | INWKGIAAMAKKLL-NH$_2$ | 1 nM |
| Mastoparan Polist. | VNWKKIGQHILSVL-NH$_2$ | 4 nM |
| Mastoparan | INLKALAALAKKIL-NH$_2$ | 0.3 nM |
| NO-30 | KRRAIGFKKLAEAVKFSAKLMGQAMAKRVK-NH$_2$ | 2 nM |
| AC-28 | IKPAKRMKFKTVCYLLVQLMHCRKMFKA-NH$_2$ | 2 nM |

TABLE 2:

Sequences of calmodulin-binding TAG peptides

| mastoparan | INLKALAALAKKIL-NH$_2$ |
|---|---|
| TAG 1 | C-EIKRAAA-INLKALAALAKKIL-NH$_2$ |
| TAG 2 | C-EIKRAAA-INLKALAALAKKIL-OH |
| TAG 3 | C-EIKRAAA-INLKALAALAKKIL-IKRKRQQ-NH$_2$ |
| TAG 4 | C-EIKRAAA-INLKALAALAKKIL-IK-OH |

Synthetic peptides derived from mastoparan that bind to calmodulin. For the TAG3 peptide, a sequence derived from melittin (which also binds to calmodulin) was incorporated in addition.

## Claims

1. A molecule comprising (i) a polypeptide ("calcium-dependent binding polypeptide") able to bind a ligand of calmodulin with a binding affinity which is dependent on the presence of calcium ions, and (ii) another polypeptide comprising a binding domain of an immunoglobulin member of a specific binding pair (sbp).

2. A molecule according to claim 1 wherein the calcium-dependent binding polypeptide is able to bind a ligand with a binding affinity with a dissociation constant ($K_d$) of 10nM or less, measured at a pH of between 6 and 9 at 20°C, in the presence of calcium ion.

3. A molecule according to claim 2 wherein said dissociation constant is 1nM or less.

4. A molecule according to any one of the preceding claims wherein the binding affinity is for a polypeptide ligand.

5. A molecule according to claim 4 wherein the polypeptide ligand is mastoparan or a polypeptide derived from mastoparan.

6. A molecule according to any one of the preceding claims wherein the calcium-dependent binding polypeptide and the ligand bind with a half-life of at least about 15 minutes.

7. A molecule according to any one of the preceding claims wherein the calcium-dependent binding polypeptide has a negative charge at about pH 8 of at least -5.

8. A molecule according to claim 7 wherein the calcium-dependent binding polypeptide has negative charge at about pH 8 of at least -10.

9. A molecule according to claim 1 wherein the calcium-dependent binding polypeptide is calmodulin, troponin C or a fragment or derivative thereof.

10. A molecule according to any one of claims 1 to 9 wherein said polypeptides are fused together via a peptide bond.

11. A molecule according to any one of claims 1 to 10 wherein the calcium-dependent binding polypeptide is labelled.

12. A molecule according to claim 11 wherein the label is fluorescence.

13. Nucleic acid comprising a nucleotide sequence encoding a molecule according to claim 10.

14. Nucleic acid according to claim 13 further comprising a promoter for expression of the molecule.

15. A host cell containing nucleic acid according to claim 13 or claim 14.

16. A method of making a molecule according to claim 10, comprising growing host cells according to claim 15 under conditions for expression of the molecule.

17. A method which comprises a step, following expression of the molecule in accordance with the method of claim 16, of isolating the molecule from the host cells.

18. A method according to claim 17 wherein the isolating involves binding of the calcium-dependent binding polypeptide to ligand.

19. A method of making a molecule comprising (i) a polypeptide ("calcium-dependent binding polypeptide") able to bind a ligand with a binding affinity with a dissociation constant of 10nM or less, this binding affinity being dependent on the presence of calcium ions, and (ii) another polypeptide, the method comprising growing host cells containing nucleic acid encoding the molecule under conditions for expression of the molecule and isolating the molecule using binding of the calcium-dependent binding polypeptide to ligand.

20. A method of isolating a molecule comprising (i) a polypeptide ("calcium-dependent binding polypeptide") able to bind a ligand of calmodulin with a binding affinity which is dependent on the presence of calcium ions, and (ii) another polypeptide, which involves binding of the calcium-dependent binding molecule to ligand.

21. A method according to claim 18, claim 19 or claim 20 which involves elution to dissociate the calcium-dependent binding molecule and ligand.

22. A method according to claim 21 wherein the elution involves use of a calcium sequestrant.

23. A method according to any one of claims 18 to 22 wherein the ligand comprises mastoparan or a polypeptide

EP 0 726 951 B1

derived from mastoparan.

24. A method according to any one of claims 18 to 23 wherein the ligand is labelled.

25. A method according to claim 24 wherein the label is fluorescence.

26. A method according to any one of claims 18 to 20 wherein the isolation involves ion exchange chromatography.

27. A method which comprises a step, following isolation of the molecule comprising a calcium-dependent binding polypeptide and another polypeptide in accordance with the method of any one of claims 17 to 26, of cleaving the calcium-dependent binding polypeptide from the molecule.

28. A method of labelling cells which comprises binding a molecule according to any one of claims 1 to 9 or comprising (i) a polypeptide ("calcium-dependent binding polypeptide") able to bind a ligand of calmodulin with a binding affinity which is dependent on the presence of calcium ions, and (ii) another polypeptide comprising a binding domain of a member of a specific binding pair (sbp) other than an immunoglobulin, via its binding domain to complementary sbp member on the surface of the cells, the calcium-dependent binding polypeptide of the molecule being labelled.

29. A method of labelling cells which comprises binding a molecule according to any one of claims 1 to 9 or comprising (i) a polypeptide ("calcium-dependent binding polypeptide") able to bind a ligand of calmodulin with a binding affinity which is dependent on the presence of calcium ions, and (ii) another polypeptide comprising a binding domain of a member of a specific binding pair (sbp) other than an immunoglobulin, via its binding domain to complementary sbp member on the surface of the cells and binding the calcium-dependent binding polypeptide to a labelled ligand.

30. A method according to claim 29 wherein the ligand comprises mastoparan or a polypeptide derived from mastoparan.

31. A method according to any one of claims 28 to 30 wherein the label is fluorescence.

32. A method which comprises sorting cells labelled by a method in accordance with claim 31 by fluorescent activated cell sorting (FACS).

33. A method which comprises imaging cells labelled by a method in accordance with any one of claims 28 to 31.

34. A multimer comprising (a) a first molecule which is a molecule according to any one of claims 1 to 9 or comprising (i) a polypeptide ("calcium-dependent binding polypeptide") able to bind a ligand of calmodulin with a binding affinity which is dependent on the presence of calcium ions, and (ii) another polypeptide comprising a binding domain of a member of a specific binding pair (sbp) other than an immunoglobulin, and (b) a second molecule which comprises a polypeptide fusion of a ligand for the calcium-dependent binding polypeptide and another polypeptide, the first and second molecules being non-covalently bound together via binding of the calcium-dependent binding polypeptide of the first molecule to the ligand.

35. A multimer according to claim 34 wherein said ligand comprises mastoparan or a polypeptide derived from mastoparan.

36. A multimer according to claim 34 or claim 35 which has a half-life of multimerisation of 15 minutes or more.

37. A band-shift assay method for determining binding affinity of said binding domain of a molecule according to any one of claims 1 to 9 or comprising (i) a polypeptide ("calcium-dependent binding polypeptide") able to bind a ligand of calmodulin with a binding affinity which is dependent on the presence of calcium ions and (ii) another polypeptide comprising a binding domain of a member of a specific binding pair (sbp) other than an immunoglobulin for a complementary sbp member of interest, the method comprising binding the calcium-dependent binding polypeptide of the molecule to a labelled ligand and comparing electrophoretic mobility of the resultant multimer in the presence and absence of the complementary sbp member.

38. A band-shift assay method for determining binding affinity of a molecule of claim 2, the binding affinity determined being that of the sbp member binding domain for complementary sbp member, the method comprising binding the calcium-dependent binding polypeptide to said ligand and comparing the electrophoretic mobility of the resultant

17

multimer in the presence and absence of said complementary sbp member.

39. A band-shift assay method according to claim 38 wherein said dissociation constant is 1nM or less.

40. A band-shift assay method according to claim 38 or claim 39 wherein the first polypeptide has a negative charge at pH 8 of at least -5.

41. A band-shift assay method according to claim 40 wherein the first polypeptide has negative charge at about pH 8 of at least -10.

42. A band-shift assay method according to any one of claims 38 to 41 wherein the first polypeptide and the ligand bind with a half-life of at least about 15 minutes.

43. A band-shift assay method according to any one of claims 38 to 42 wherein the calcium-dependent binding polypeptide is calmodulin, troponin C or a fragment or derivative thereof.

44. A band-shift assay method according to any one of claims 38 to 43 wherein the ligand comprises a label and the label is fluorescence.

45. A molecule comprising a calmodulin-binding polypeptide, which is mastoparan or a polypeptide derived from mastoparan, and another polypeptide, which comprises a binding domain of a member of a specific binding pair (sbp).

46. A molecule according to claim 45 wherein the calmodulin-binding polypeptide has an amino acid sequence which is

$$INLKALAALAKKIL-CONH_2,$$

$$C-EIKRAAA-INLKALAALAKKIL-COOH,$$

$$C-EIKRAAA-INLKALAALAKKIL-CONH_2,$$

$$C-EIKRAAA-INLKALAALAKKIL-IKRKRQQ-CONH_2,$$

$$C-EIKRAAA-INLKALAALAKKIL-IK-COOH$$

or a derivative of any of these.

47. A molecule according to claim 45 or 46 wherein the binding domain is an immunoglobulin binding domain.

48. A molecule according to any one of claims 45 to 47 wherein the calmodulin-binding polypeptide and the other polypeptide are fused together via a peptide bond.

49. A molecule according to any one of claims 45 to 48 wherein the calmodulin-binding polypeptide is labelled.

50. A molecule according to claim 49 wherein the label is fluorescence.

51. Nucleic acid with a nucleotide sequence encoding a molecule according to claim 48.

52. Nucleic acid according to claim 51 further comprising a promoter for expression of the molecule.

53. A host cell containing a nucleic acid according to claim 51 or claim 52.

54. A method of making a molecule comprising a calmodulin-binding polypeptide and another polypeptide, the method comprising growing host cells according to claim 53 under conditions for expression of the molecule.

55. A method which comprises a step, following expression of the molecule in accordance with the method of claim 54, of isolating the molecule from the host cells.

**56.** A method according to claim 55 wherein the isolating involves binding of the calmodulin-binding polypeptide to calmodulin, troponin C or a fragment or derivative thereof.

**57.** A method of isolating a molecule according to any one of claims 45 to 50 which involves binding of the calmodulin-binding polypeptide to calmodulin, troponin C or a fragment or derivative thereof.

**58.** A method according to claim 56 or claim 57 which involves elution to dissociate the calmodulin, troponin C, fragment or derivative and the calmodulin-binding molecule.

**59.** A method according to claim 58 wherein the elution involves use of a calcium sequestrant.

**60.** A method which comprises a step, following isolation of the molecule comprising a calmodulin-binding polypeptide and another polypeptide in accordance with the method of any one of claims 55 to 59, of cleaving the calmodulin-binding polypeptide from the molecule.

**61.** A method of labelling cells which comprises binding a molecule according to any one of claims 45 to 47 via its binding domain to complementary sbp member on the surface of the cells, the calmodulin-binding polypeptide of the molecule being labelled.

**62.** A method of labelling cells which comprises binding a molecule according to any one of claims 45 to 47 via its binding domain to complementary sbp member on the surface of the cells and binding the calmodulin-binding polypeptide to labelled calmodulin, troponin C or a fragment or derivative thereof.

**63.** A method according to claim 61 or claim 62 wherein the label is fluorescence.

**64.** A method which comprises sorting cells labelled by a method in accordance with claim 63 by fluorescent activated cell sorting (FACS).

**65.** A method which comprises imaging cells labelled by a method in accordance with claim 61 or claim 62.

**66.** A band-shift assay method for determining binding affinity of the binding domain of a molecule according to any one of claims 45 to 47 for a complementary sbp member of interest, the method comprising binding the calmodulin-binding polypeptide of the molecule to labelled calmodulin, troponin C or a fragment or derivative thereof, and comparing electrophoretic mobility of the resultant multimer in the presence and absence of the complementary sbp member.

**67.** A band-shift assay according to claim 66 wherein the label is fluorescence.

**Patentansprüche**

**1.** Molekül, das (i) ein Polypeptid ("kalziumabhängiges Bindungspolypeptid"), das zur Bindung eines Calmodulin-Liganden mit einer Bindungsaffinität fähig ist, die von der Gegenwart von Kalziumionen abhängig ist, sowie (ii) ein weiteres Polypeptid umfaßt, das eine Bindedomäne eines Immunglobulinelements eines spezifischen Bindungspaares (sbp) umfaßt.

**2.** Molekül nach Anspruch 1, worin das kalziumabhängige Bindungspolypeptid einen Liganden mit einer Bindungsaffinität mit einer Dissoziationskonstante ($K_d$) von 10 nM oder weniger, gemessen bei einem pH zwischen 6 und 9 bei 20 °C, in Gegenwart von Kalziumionen binden kann.

**3.** Molekül nach Anspruch 2, worin die Dissoziationskonstante 1 nM oder weniger beträgt.

**4.** Molekül nach einem der vorangegangenen Ansprüche, worin die Bindungsaffinität für einen Polypeptidliganden besteht.

**5.** Molekül nach Anspruch 4, worin der Polypeptid-Ligand Mastoparan oder ein von Mastoparan abgeleitetes Polypeptid ist.

**6.** Molekül nach einem der vorangegangenen Ansprüche, worin sich das kalziumabhängige Bindungspolypeptid und der Ligand mit einer Halbwertszeit von zumindest etwa 15 min binden.

**7.** Molekül nach einem der vorangegangenen Ansprüche, worin das kalziumabhängige Bindungspolypeptid eine negative Ladung bei etwa pH 8 von zumindest -5 aufweist.

**8.** Molekül nach Anspruch 7, worin das kalziumabhängige Bindungspolypeptid eine negative Ladung bei etwa pH 8 von zumindest -10 aufweist.

**9.** Molekül nach Anspruch 1, worin das kalziumabhängige Bindungspolypeptid Calmodulin, Troponin C oder ein Fragment oder Derivat davon ist.

**10.** Molekül nach einem der Ansprüche 1 bis 9, worin die Polypeptide über eine Peptidbindung fusioniert sind.

**11.** Molekül nach einem der Ansprüche 1 bis 10, worin das kalziumabhängige Bindungspolypeptid markiert ist.

**12.** Molekül nach Anspruch 11, worin die Markierung Fluoreszenz ist.

**13.** Nukleinsäure, die eine Nukleotidsequenz umfaßt, die für ein Molekül nach Anspruch 10 kodiert.

**14.** Nukleinsäure nach Anspruch 13, die weiters einen Promotor zur Expression des Moleküls umfaßt.

**15.** Wirtszelle, die Nukleinsäure nach Anspruch 13 oder Anspruch 14 enthält.

**16.** Verfahren zur Herstellung eines Moleküls nach Anspruch 10, welches das Züchten von Wirtszellen nach Anspruch 15 unter Bedingungen zur Expression des Moleküls umfaßt.

**17.** Verfahren, das nach der Expression des Moleküls nach dem Verfahren aus Anspruch 16 einen Schritt des Isolierens des Moleküls aus den Wirtszellen umfaßt.

**18.** Verfahren nach Anspruch 17, worin das Isolieren die Bindung des kalziumabhängigen Bindungspolypeptids an Liganden umfaßt.

**19.** Verfahren zur Herstellung eines Moleküls, das (i) ein Polypeptid ("kalziumabhängiges Bindungspolypeptid"), das zur Bindung eines Liganden mit einer Bindungsaffinität mit einer Dissoziationskonstanten von 10 nM oder weniger fähig ist, wobei diese Bindungsaffinität von der Gegenwart von Kalziumionen abhängig ist, sowie (ii) ein weiteres Polypeptid umfaßt, wobei das Verfahren das Züchten von Wirtszellen, die für das Molekül kodierende Nukleinsäure enthalten, unter Bedingungen zur Expression des Moleküls sowie das Isolieren des Moleküls unter Nutzung der Bindung des kalziumabhängigen Bindungspolypeptids an Liganden umfaßt.

**20.** Verfahren zum Isolieren eine Moleküls, das (i) ein Polypeptid ("kalziumabhängiges Bindungspolypeptid"), das zur Bindung eines Calmodulin-Liganden mit einer Bindungsaffinität fähig ist, die von der Gegenwart von Kalziumionen abhängig ist, sowie (ii) ein weiteres Polypeptid umfaßt, umfassend die Bindung des kalziumabhängigen Bindemoleküls an Liganden.

**21.** Verfahren nach Anspruch 18, 19 oder 20, welches das Eluieren zum Dissoziieren des kalziumabhängigen Bindemoleküls vom Liganden umfaßt.

**22.** Verfahren nach Anspruch 21, worin das Eluieren die Verwendung eines Kalziumsequestranten umfaßt.

**23.** Verfahren nach einem der Ansprüche 18 bis 22, worin der Ligand Mastoparan oder ein von Mastoparan abgeleitetes Polypeptid umfaßt.

**24.** Verfahren nach einem der Ansprüche 18 bis 23, worin der Ligand markiert ist.

**25.** Verfahren nach Anspruch 24, worin die Markierung Fluoreszenz ist.

**26.** Verfahren nach einem der Ansprüche 18 bis 20, worin das Isolieren ionenaustauschchromatographie umfaßt.

27. Verfahren, welches nach dem Isolieren des ein kalziumabhängiges Bindungspolypeptid und ein weiteres Polypeptid umfassenden Moleküls gemäß dem Verfahren nach einem der Ansprüche 17 bis 26 einen Schritt des Abspaltens des kalziumabhängigen Bindungspolypeptids vom Molekül umfaßt.

28. Verfahren zum Markieren von Zellen, welches die Bindung eines Moleküls nach einem der Ansprüche 1 bis 9 oder eines Moleküls, das (i) ein Polypeptid ("kalziumabhängiges Bindungspolypeptid"), das zur Bindung eines Calmodulin-Liganden mit einer Bindungsaffinität fähig ist, die von der Gegenwart von Kalziumionen abhängig ist, sowie (ii) ein weiteres Polypeptid umfaßt, das eine Bindedomäne eines Elements eines spezifischen Bindungspaares (sbp) mit Ausnahme eines Immunglobulins umfaßt, über seine Bindedomäne an komplementäres sbp-Element an der Oberfläche der Zellen umfaßt, wobei das kalziumabhängige Bindungspolypeptid des Moleküls markiert ist.

29. Verfahren zum Markieren von Zellen, welches die Bindung eines Moleküls nach einem der Ansprüche 1 bis 9 oder eines Moleküls, das (i) ein Polypeptid ("kalziumabhängiges Bindungspolypeptid"), das zur Bindung eines Calmodulin-Liganden mit einer Bindungsaffinität fähig ist, die von der Gegenwart von Kalziumionen abhängig ist, sowie (ii) ein weiteres Polypeptid umfaßt, das eine Bindedomäne eines Elements eines spezifischen Bindungspaares (sbp) mit Ausnahme eines Immunglobulins umfaßt, über seine Bindedomäne an komplementäres sbp-Element an der Oberfläche der Zellen, sowie die Bindung des kalziumabhängigen Bindungspolypeptids an einen markierten Liganden umfaßt.

30. Verfahren nach Anspruch 29, worin der Ligand Mastoparan oder ein von Mastoparan abgeleitetes Polypeptid umfaßt.

31. Verfahren nach einem der Ansprüche 28 bis 30, worin die Markierung Fluoreszenz ist.

32. Verfahren, welches das Sortieren von gemäß einem Verfahren nach Anspruch 31 markierten Zellen durch fluoreszenzaktivierte Zellsortierung (FACS) umfaßt.

33. Verfahren, welches das Abbilden von gemäß einem Verfahren nach einem der Ansprüche 28 bis 31 markierten Zellen umfaßt.

34. Multimer, das umfaßt: (a) ein erstes Molekül, das ein Molekül nach einem der Ansprüche 1 bis 9 oder ein Molekül ist, das (i) ein Polypeptid ("kalziumabhängiges Bindungspolypeptid"), das zur Bindung eines Calmodulin-Liganden mit einer Bindungsaffinität fähig ist, die von der Gegenwart von Kalziumionen abhängig ist, sowie (ii) ein weiteres Polypeptid umfaßt, das eine Bindedomäne eines Elements eines spezifischen Bindungspaares (sbp) mit Ausnahme von Immunglobulin umfaßt, und (b) ein zweites Molekül, das eine Polypeptidfusion aus einem Liganden für das kalziumabhängige Bindungspolypeptid und einem weiteren Polypeptid umfaßt, wobei das erste und das zweite Molekül über die Bindung des kalziumabhängigen Bindungspolypeptids des ersten Moleküls an den Liganden nicht-kovalent miteinander verbunden sind.

35. Multimer nach Anspruch 34, worin der Ligand Mastoparan oder ein von Mastoparan abgeleitetes Polypeptid umfaßt.

36. Multimer nach Anspruch 34 oder 35, das eine Multimerisations-Halbwertszeit von 15 min oder mehr aufweist.

37. Bandenverschiebungs-Assayverfahren zum Bestimmen der Bindungsaffinität der Bindedomäne eines Moleküls nach einem der Ansprüche 1 bis 9 oder eines Moleküls, das (i) ein Polypeptid ("kalziumabhängiges Bindungspolypeptid"), das zur Bindung eines Calmodulin-Liganden mit einer Bindungsaffinität fähig ist, die von der Gegenwart von Kalziumionen abhängig ist, sowie (ii) ein weiteres Polypeptid umfaßt, das eine Bindedomäne eines Elements eines spezifischen Bindungspaares (sbp) mit Ausnahme eines Immunglobulins für ein komplementäres sbp-Element von Interesse umfaßt, wobei das Verfahren die Bindung des kalziumabhängigen Bindungspolypeptids des Moleküls an einen markierten Liganden und das Vergleichen der elektrophoretischen Mobilität des resultierenden Multimers in Gegenwart und Abwesenheit des komplementären sbp-Elements umfaßt.

38. Bandenverschiebungs-Assayverfahren zum Bestimmen der Bindungsaffinität eines Moleküls nach Anspruch 2, wobei die bestimmte Bindungaffinität jene der sbp-Element-Bindedomäne für komplementäres sbp-Element ist, wobei das Verfahren die Bindung das kalziumabhängigen Bindungspolypeptids an den Liganden und das Vergleichen der elektrophoretischen Mobilität des resultierenden Multimers in Gegenwart und Abwesenheit des komplementären sbp-Elements umfaßt.

**39.** Bandenverschiebungs-Assayverfahren nach Anspruch 38, worin die Dissoziationskonstante 1 nM oder weniger beträgt.

**40.** Bandenverschiebungs-Assayverfahren nach Anspruch 38 oder 39, worin das erste Polypeptid eine negative Ladung bei pH 8 von zumindest -5 aufweist.

**41.** Bandenverschiebungs-Assayverfahren nach Anspruch 40, worin das erste Polypeptid eine negative Ladung bei etwa pH 8 von zumindest -10 aufweist.

**42.** Bandenverschiebungs-Assayverfahren nach einem der Ansprüche 38 bis 41, worin sich das erste Polypeptid und der Ligand mit einer Halbswertszeit von zumindest etwa 15 min binden.

**43.** Bandenverschiebungs-Assayverfahren nach einem der Ansprüche 38 bis 42, worin das kalziumabhängige Bindungspolypeptid Calmodulin, Troponin C oder ein Fragment oder Derivat davon ist.

**44.** Bandenverschiebungs-Assayverfahren nach einem der Ansprüche 38 bis 43, worin der Ligand eine Markierung umfaßt und die Markierung Fluoreszenz ist.

**45.** Molekül, das ein Calmodulin-Bindungspolypeptid, das Mastoparan oder ein von Mastoparan abgeleitetes Polypeptid ist, sowie ein weiteres Polypeptid umfaßt, welches eine Bindedomäne eines Elements eines spezifischen Bindungspaares (sbp) umfaßt.

**46.** Molekül nach Anspruch 45, worin das Calmodulin-Bindungspolypeptid eine Aminosäuresequenz, die

$$INLKALAALAKKIL-CONH_2,$$

$$C-EIKRAAA-INLKALAALAKKIL-COOH,$$

$$C-EIKRAAA-INLKALAALAKKIL-CONH_2,$$

$$C-EIKRAAA-INLKALAALAKKIL-IKRKRQQ-CONH_2,$$

$$C-EIKRAAA-INLKALAALAKKIL-IK-COOH$$

ist, oder Derivat irgendeiner davon aufweist.

**47.** Molekül nach Anspruch 45 oder 46, worin die Bindedomäne eine Immunglobulin-Bindedomäne ist.

**48.** Molekül nach einem der Ansprüche 45 bis 47, worin das Calmodulin-Bindungspolypeptid und das andere Polypeptid über eine Peptidbindung miteinander fusioniert sind.

**49.** Molekül nach einem der Ansprüche 45 bis 48, worin das Calmodulin-Bindungspolypeptid markiert ist.

**50.** Molekül nach Anspruch 49, worin die Markierung Fluoreszenz ist.

**51.** Nukleinsäure mit einer Nukleotidsequenz, die für ein Molekül nach Anspruch 48 kodiert.

**52.** Nukleinsäure nach Anspruch 51, die weiters einen Promotor zur Expression des Moleküls umfaßt.

**53.** Wirtszelle, die eine Nukleinsäure nach Anspruch 51 oder 52 enthält.

**54.** Verfahren zur Herstellung eines Moleküls, das ein Calmodulin-Bindungspolypeptid und ein anderes Polypeptid umfaßt, wobei das Verfahren das Züchten von Wirtszellen nach Anspruch 53 unter Bedingungen zur Expression des Moleküls umfaßt.

**55.** Verfahren, das nach der Expression des Moleküls gemäß dem Verfahren nach Anspruch 54 einen Schritt des Isolierens des Moleküls aus den Wirtszellen umfaßt.

56. Verfahren nach Anspruch 55, worin das Isolieren die Bindung des Calmodulin-Bindungspolypeptids an Calmodulin, Troponin C oder ein Fragment oder Derivat davon umfaßt.

57. Verfahren zum Isolieren eines Moleküls nach einem der Ansprüche 45 bis 50, welches das Binden des Calmodulin-Bindungspolypeptids an Calmodulin, Troponin C oder ein Fragment oder Derivat davon umfaßt.

58. Verfahren nach Anspruch 56 oder 57, welches das Eluieren zum Dissoziieren des Calmodulins, Troponins C, Fragments oder Derivats und des Calmodulin-Bindemoleküls umfaßt.

59. Verfahren nach Anspruch 58, worin das Eluieren die Verwendung eines Kalziumsequestranten umfaßt.

60. Verfahren, welches nach dem Isolieren des ein Calmodulin-Bindungspolypeptid und ein weiteres Polypeptid umfassenden Moleküls gemäß dem Verfahren nach einem der Ansprüche 55 bis 59 einen Schritt des Abspaltens des Calmodulin-Bindungspolypeptids vom Molekül umfaßt.

61. Verfahren zum Markieren von Zellen, welches die Bindung eines Moleküls nach einem der Ansprüche 45 bis 47 über seine Bindedomäne an komplementäres sbp-Element an der Oberfläche der Zellen umfaßt, wobei das Calmodulin-Bindungspolypeptid des Moleküls markiert ist.

62. Verfahren zum Markieren von Zellen, welches die Bindung eines Moleküls nach einem der Ansprüche 45 bis 47 über seine Bindedomäne an komplementäres sbp-Element an der Oberfläche der Zellen und die Bindung des Calmodulin-Bindungspolypeptids an markiertes Calmodulin, Troponin C oder ein Fragment oder Derivat davon umfaßt.

63. Verfahren nach Anspruch 61 oder 62, worin die Markierung Fluoreszenz ist.

64. Verfahren, welches das Sortieren von nach einem Verfahren nach Anspruch 63 markierten Zellen durch fluoreszenzaktivierte Zellsortierung (FACS) umfaßt.

65. Verfahren, welches das Abbilden von Zellen umfaßt, die gemäß einem Verfahren nach Anspruch 61 oder Anspruch 62 markiert sind.

66. Bandenverschiebungs-Assayverfahren zum Bestimmen der Bindungsaffinität der Bindedomäne eines Moleküls nach einem der Ansprüche 45 bis 47 für ein komplementäres sbp-Element von Interesse, wobei das Verfahren die Bindung des Calmodulin-Bindungspolypeptids des Moleküls an markiertes Calmodulin, Troponin C oder ein Fragment oder Derivat davon und das Vergleichen der elektrophoretischen Mobilität des resultierenden Multimers in Gegenwart und Abwesenheit des komplementären sbp-Elements umfaßt.

67. Bandenverschiebungs-Assayverfahren nach Anspruch 66, worin die Markierung Fluoreszenz ist.

## Revendications

1. Molécule comprenant (i) un polypeptide ("polypeptide de liaison dépendant du calcium") capable de se lier à un ligand de calmoduline avec une affinité de liaison qui dépend de la présence d'ions calcium, et (ii) un autre polypeptide comprenant un domaine de liaison d'un membre d'immunoglobuline d'une paire de liaison spécifique (sbp).

2. Molécule selon la revendication 1 où le polypeptide de liaison dépendant du calcium est capable de se lier à un ligand avec une affinité de liaison avec une constante de dissociation ($K_d$) de 10 nM ou moins, mesurée à un pH entre 6 et 9 à 20°C, en présence d'un ion calcium.

3. Molécule selon la revendication 2 où ladite constante de dissociation vaut 1 nM ou moins.

4. Molécule selon l'une quelconque des revendications précédentes où l'affinité de liaison est pour un ligand de polypeptide.

**5.** Molécule selon la revendication 4 où le ligand de polypeptide est du mastoparan ou un polypeptide dérivé du mastoparan.

**6.** Molécule selon l'une quelconque des revendications précédentes où le polypeptide de liaison dépendant du calcium et le ligand se lient avec une demi-vie d'au moins environ 15 minutes.

**7.** Molécule selon l'une quelconque des revendications précédentes où le polypeptide de liaison dépendant du calcium a une charge négative à environ pH 8 d'au moins -5.

**8.** Molécule selon la revendication 7 où le polypeptide de liaison dépendant du calcium a une charge négative à environ pH 8 d'au moins -10.

**9.** Molécule selon la revendication 1 où le polypeptide de liaison dépendant du calcium est la calmoduline, la troponine C ou un fragment ou dérivé de celles-ci.

**10.** Molécule selon l'une quelconque des revendications 1 à 9 où lesdits polypeptides sont fusionnés ensemble via une liaison peptide.

**11.** Molécule selon l'une quelconque des revendications 1 à 10 où le peptide de liaison dépendant du calcium est marqué.

**12.** Molécule selon la revendication 11 où le marqueur est une fluorescence.

**13.** Acide nucléique comprenant une séquence de nucléotides encodant une molécule selon la revendication 10.

**14.** Acide nucléique selon la revendication 13 comprenant de plus un promoteur pour une expression de la molécule.

**15.** Cellule hôte contenant un acide nucléique selon la revendication 13 ou la revendication 14.

**16.** Méthode de fabrication d'une molécule selon la revendication 10, comprenant la croissance de cellules hôtes selon la revendication 15 dans des conditions pour une expression de la molécule.

**17.** Méthode qui comprend une étape, suivant l'expression de la molécule selon la méthode de la revendication 16, d'isolement de la molécule des cellules hôtes.

**18.** Méthode selon la revendication 17 où l'isolement implique la liaison du polypeptide de liaison dépendant du calcium à un ligand.

**19.** Méthode de fabrication d'une molécule comprenant (i) un polypeptide ("polypeptide de liaison dépendant du calcium") capable de se lier à un ligand avec une affinité élevée avec une constante de dissociation de 10 nM ou moins, cette affinité de liaison dépendant de la présence d'ions calcium, et (ii) un autre polypeptide, la méthode comprenant la croissance des cellules hôtes contenant un acide nucléique encodant la molécule dans des conditions pour une expression de la molécule et l'isolement de la molécule en utilisant la liaison du polypeptide de liaison dépendant du calcium au ligand.

**20.** Méthode d'isolement d'une molécule comprenant (i) un polypeptide ("polypeptide de liaison dépendant du calcium") capable de se lier à un ligand de calmoduline avec une affinité de liaison qui dépend de la présence d'ions calcium, et (ii) un autre polypeptide, qui implique la liaison de la molécule de liaison dépendant du calcium à un ligand.

**21.** Méthode selon la revendication 18, la revendication 19 ou la revendication 20, qui implique une élution pour dissocier la molécule de liaison dépendant du calcium et le ligand.

**22.** Méthode selon la revendication 21 où l'élution implique l'utilisation d'un séquestrant de calcium.

**23.** Méthode selon l'une quelconque des revendications 18 à 22 où le ligand comprend du mastoparan ou un polypeptide dérivé de mastoparan.

**EP 0 726 951 B1**

24. Méthode selon l'une quelconque des revendications 18 à 23 où le ligand est marqué.

25. Méthode selon la revendication 24 où le marqueur est une fluorescence.

26. Méthode selon l'une quelconque des revendications 18 à 20 où l'isolement implique une chromatographie d'échange d'ions.

27. Méthode qui comprend une étape, suivant l'isolement de la molécule comprenant un polypeptide de liaison dépendant du calcium et un autre polypeptide selon la méthode de l'une quelconque des revendications 17 à 26, de clivage du polypeptide de liaison dépendant du calcium de la molécule.

28. Méthode de marquage de cellules qui comprend la liaison d'une molécule selon l'une quelconque des revendications 1 à 9 ou comprenant (i) un polypeptide ("polypeptide de liaison dépendant du calcium") capable de se lier à un ligand de calmoduline avec une affinité de liaison qui dépend de la présence d'ions calcium, et (ii) un autre polypeptide comprenant un domaine de liaison d'un membre d'une paire de liaison spécifique (sbp) différent d'une immunoglobuline, via son domaine de liaison au membre sbp complémentaire sur la surface des cellules, le polypeptide de liaison dépendant du calcium de la molécule étant marqué.

29. Méthode de marquage de cellules qui comprend la liaison d'une molécule selon l'une quelconque des revendications 1 à 9 ou comprenant (i) un polypeptide ("polypeptide de liaison dépendant du calcium") capable de se lier à un ligand de calmoduline avec une affinité de liaison qui dépend de la présence d'ions calcium, et (ii) un autre polypeptide comprenant un domaine de liaison d'un membre d'une paire de liaison spécifique (sbp) différent d'une immunoglobuline, via son domaine de liaison à un membre de sbp complémentaire sur la surface des cellules et la liaison du polypeptide de liaison dépendant du calcium à un ligand marqué.

30. Méthode selon la revendication 29 où le ligand comprend du mastoparan ou un polypeptide dérivé du mastoparan.

31. Méthode selon l'une quelconque des revendications 28 à 30 où le marqueur est une fluorescence.

32. Méthode qui comprend le tri des cellules marquées par une méthode selon la revendication 31 par un tri des cellules activées fluorescentes (FACS).

33. Méthode qui comprend la représentation par une image des cellules marquées par une méthode selon l'une quelconque des revendications 28 à 31.

34. Multimère comprenant (a) une première molécule qui est une molécule selon l'une quelconque des revendications 1 à 9 ou comprenant (i) un polypeptide ("polypeptide de liaison dépendant du calcium") capable de se lier à un ligand de calmoduline avec une affinité de liaison qui dépend de la présence d'ions calcium, et (ii) un autre polypeptide comprenant un domaine de liaison d'un membre d'une paire de liaison spécifique (sbp) différent d'une immunoglobuline, et (b) une seconde molécule qui comprend une fusion de polypeptide d'un ligand pour le polypeptide de liaison dépendant du calcium et un autre polypeptide, les première et seconde molécules étant liées de façon non covalente ensemble via une liaison du polypeptide de liaison dépendant du calcium de la première molécule au ligand.

35. Multimère selon la revendication 34 où ledit ligand comprend du mastoparan ou un polypeptide dérivé de mastoparan.

36. Multimère selon la revendication 34 ou 35 qui a une demi-vie de multimérisation de 15 minutes ou plus.

37. Méthode de dosage d'un déplacement de bande pour déterminer une affinité de liaison dudit domaine de liaison d'une molécule selon l'une quelconque des revendications 1 à 9 ou comprenant (i) un polypeptide ("polypeptide de liaison dépendant du calcium") capable de se lier à un ligand de calmoduline avec une affinité de liaison qui dépend de la présence d'ions calcium, et (ii) un autre polypeptide comprenant un domaine de liaison d'un membre d'une paire de liaison spécifique (sbp) différent d'une immunoglobuline, pour un membre sbp complémentaire d'intérêt, la méthode comprenant la liaison du polypeptide de liaison dépendant du calcium de la molécule à un ligand marqué et la comparaison de la mobilité électrophorétique du multimère résultant, en présence et en l'absence du membre sbp complémentaire.

**38.** Méthode de dosage d'un déplacement de bande pour déterminer une affinité de liaison d'une molécule selon la revendication 2, l'affinité de liaison déterminée étant celle du domaine de liaison du membre sbp pour le membre sbp complémentaire, la méthode comprenant la liaison du polypeptide de liaison dépendant du calcium audit ligand et la comparaison de la mobilité électrophorétique du dimère résultant, en présence et en l'absence dudit membre sbp complémentaire.

**39.** Méthode de dosage d'un déplacement de bande selon la revendication 38 où ladite constante de dissociation vaut 1 nM ou moins.

**40.** Méthode de dosage d'un déplacement de bande selon la revendication 38 ou la revendication 39 où le premier polypeptide a une charge négative à pH 8 d'au moins -5.

**41.** Méthode de dosage d'un déplacement de bande selon la revendication 40 où le premier polypeptide a une charge négative à environ pH 8 d'au moins -10.

**42.** Méthode de dosage d'un déplacement de bande selon l'une quelconque des revendications 38 à 41 où le premier polypeptide et le ligand se lient avec une demi-vie d'au moins environ 15 minutes.

**43.** Méthode de dosage d'un déplacement de bande selon l'une quelconque des revendications 38 à 42 où le polypeptide de liaison dépendant du calcium est la calmoduline, la troponine C ou un fragment ou un dérivé de celle-ci.

**44.** Méthode de dosage d'un déplacement de bande selon l'une quelconque des revendications 38 à 43 où le ligand comprend un marqueur et le marqueur est une fluorescence.

**45.** Molécule comprenant un polypeptide liant la calmoduline, qui est le mastoparan ou un polypeptide dérivé du mastoparan, et un autre polypeptide, qui comprend un domaine de liaison d'un membre d'une paire de liaison spécifique (sbp).

**46.** Molécule selon la revendication 45 où le polypeptide liant la calmoduline a une séquence d'acides aminés qui est

$$INLKALAALAKKIL-CONH_2,$$

$$C-EIKRAAA-INLKALAALAKKIL-COOH,$$

$$C-EIKRAAA-INLKALAALAKKIL-CONH_2,$$

$$C-EIKRAAA-INLKALAALAKKIL-IKRKRQQ-CONH_2,$$

$$C-EIKRAAA-INLKALAALAKKIL-IK-COOH$$

ou un dérivé de l'une de celle-ci.

**47.** Molécule selon la revendication 45 ou 46 où le domaine de liaison est un domaine de liaison d'immunoglobuline.

**48.** Molécule selon l'une quelconque des revendications 45 à 47 où le polypeptide liant la calmoduline et l'autre polypeptide sont fusionnés ensemble via une liaison peptide.

**49.** Molécule selon l'une quelconque des revendications 45 à 48 où le polypeptide liant la calmoduline est marqué.

**50.** Méthode selon la revendication 49 où le marqueur est une fluorescence.

**51.** Acide nucléique avec une séquence de nucléotides encodant une molécule selon la revendication 48.

**52.** Acide nucléique selon la revendication 51 comprenant de plus un promoteur pour une expression de la molécule.

**53.** Cellule hôte contenant un acide nucléique selon la revendication 51 ou la revendication 52.

**54.** Méthode de fabrication d'une molécule comprenant un polypeptide liant la calmoduline et un autre polypeptide, la méthode comprenant la croissance de cellules hôtes selon la revendication 53 dans des conditions pour une expression de la molécule.

**55.** Méthode qui comprend une étape, suivant l'expression de la molécule selon la méthode de la revendication 54, d'isolement de la molécule des cellules hôtes.

**56.** Méthode selon la revendication 55 où l'isolement implique la liaison du polypeptide de liaison de la calmoduline à de la calmoduline, de la troponine C ou un de leur fragment ou dérivé.

**57.** Méthode d'isolement d'une molécule selon l'une quelconque des revendications 45 à 50 qui implique la liaison d'un polypeptide liant la calmoduline à la calmoduline, la troponine C ou un de leur fragment ou dérivé.

**58.** Méthode selon la revendication 56 ou 57 qui implique une élution pour dissocier la calmoduline, la troponine C, leur fragment ou dérivé et la molécule liant la calmoduline.

**59.** Méthode selon la revendication 58 où l'élution implique l'utilisation d'un séquestrant de calcium.

**60.** Méthode qui comprend une étape, suivant l'isolement de la molécule comprenant un polypeptide liant la calmoduline et un autre polypeptide selon la méthode d'une quelconque des revendications 55 à 59, de clivage du polypeptide liant la calmoduline de la molécule.

**61.** Méthode de marquage de cellules qui comprend la liaison d'une molécule selon l'une quelconque des revendications 45 à 47 via son domaine de liaison à un membre de sbp complémentaire sur la surface des cellules, le polypeptide liant la calmoduline de la molécule étant marqué.

**62.** Méthode de marquage de cellules qui comprend la liaison d'une molécule selon l'une quelconque des revendications 45 à 47 via son domaine de liaison à un membre de sbp complémentaire sur la surface des cellules, et la liaison du polypeptide liant la calmoduline à la calmoduline marquée, la troponine C marquée ou un de leur fragment ou dérivé marqué.

**63.** Méthode selon la revendication 61 ou la revendication 62 où le marqueur est une fluorescence.

**64.** Méthode qui comprend le tri des cellules marquées par une méthode selon la revendication 23 par un tri de cellules activées fluorescentes (FACS).

**65.** Méthode qui comprend la représentation par une image des cellules marquées par une méthode selon la revendication 61 ou la revendication 62.

**66.** Méthode de dosage d'un déplacement de bande pour déterminer l'affinité de liaison du domaine de liaison d'une molécule selon l'une quelconque des revendications 45 à 47 pour un membre de sbp complémentaire d'intérêt, la méthode comprenant la liaison du polypeptide liant la calmoduline de la molécule à la calmoduline marquée, la troponine C marquée ou un de leur fragment ou dérivé marqué, et la comparaison de la mobilité électrophorétique du multimère résultant en la présence et en l'absence du membre de sbp complémentaire.

# Fig.1.

antigen binding site

N

V_L

C

tag

EP 0 726 951 B1

V_H

**HyHEL10**

**Calmodulin**

# Fig.2.

**PelB leader**

<u>    L   L   L   A   A   Q   P   A   M   A   Q   V   Q   L   Q   V   D</u>

...TTATTACTCGC<u>GGCCCAGCCGGCC</u>ATGGCCCAGGTGCAG<u>CTGCAGGTCGAC</u>
                   SfiI                                    PstI   SalI

**calmodulin**

L   E   I   K   R   A   A   <u>A   D   Q   L   T   E   E   Q</u>

<u>CTCGAG</u>GATCAACGG<u>GCGGCCGC</u>TGACCAACTGACAGAAGAGCAG......
XhoI                NotI

EP 0 726 951 B1

# Fig.3.

1  2  3  4  5  6

— 69.8
— 43.3
— 28.3
— 18.1
— 15.4

# Fig.5.

1 2 3 4 5 6 7 8

← scFv-Cal

PERI
PURE
PURE + CEA
PURE + HEL
PERI
PURE
PURE + HEL
PURE + CEA

anti-CEA     anti-HEL

# Fig.4.

competition time

| 0 | 1/2 | 1 | 2 | 4 | hrs |

CAL/TAG-3

EP 0 726 951 B1

# Fig.6.

## Fig.7.

## Fig.8a.

EP 0 726 951 B1

# Fig.8b.

# Fig.11.

Fig.9.

FLUORESCENTLY STAINED GEL

COOMASSIE BLUE STAINED GEL

# Fig.10.

```
       stop
rbs
      L — polylinker — tag
```

GCATGCAAAT TCTATTTCAA GGAGACAGTC ATAATGAAAT ACCTATTGCC
SphI


TACGGCAGCC GCTGGATTGT TATTACTCGC GGCCCAGCCG GCCATGGCCC
                                      SfiI


                                              ------
AGGTGCAGCT GCAGGTCGAC CTCGAGATCA AACGGGCGGC CGCAATCAAC
       PstI    SalI   XhoI                 NotI


---------calm tag ------------------- stop -
CTGAAAGCTC TAGCCGCGCT GGCCAAAAAA ATCCTGTAAT AAGAATTC
                                              EcoRI

37

# Fig.12.

Calmodulin sequence

```
M  A  D  Q  L  T  E  E  Q  I  A  E  F  K  E  A  F  S  L  F
D  K  D  G  D  G  T  I  T  T  K  E  L  G  T  V  M  R  S  L
G  Q  N  P  T  E  A  E  L  Q  D  M  I  N  E  V  D  A  D  G
N  G  T  I  D  F  P  E  F  L  T  M  M  A  R  K  M  K  D  T
D  S  E  E  E  I  R  E  A  F  R  V  F  D  K  D  G  N  G  Y
I  S  A  A  E  L  R  H  V  M  T  N  L  G  E  K  L  T  D  E
E  V  D  E  M  I  R  E  A  D  I  D  G  D  G  Q  V  N  Y  E
E  F  V  Q  M  M  T  A  K
```